# EUROPEAN PATENT APPLICATION

(11) **EP 4 825 394 A1**
(43) Date of publication of application: **21.10.2026**
(21) Application number: 26168888.1
(22) Date of filing: 30.03.2026
(51) Int. Cl.: A61K 39/00, A61K 9/14, A61K 39/12

(54) **PHASE-SEPARATING PEPTIDE COACERVATES FOR NUCLEIC ACID TRANSFECTION AND MRNA VACCINE DELIVERY**

(30) Priority: 14.04.2025 US 202563788315 P
(71) Applicant: The Chinese University of Hong Kong, Shatin, New Territories Hong Kong (CN); The University of Hong Kong, Hong Kong Special Administrative Region (CN)
(72) Inventor: XIA, Jiang, Hong Kong (CN); XU, Zhiyi, Hong Kong (CN); HUANG, Jiandong, Hong Kong (CN); KUWENTRAI, Chaiyaporn, Hong Kong (CN)
(74) Representative: WSL Patentanwälte Partnerschaft mbB

(57) **Abstract**

The subject invention pertains to novel phase-separating peptide-based nucleic acid formulations and methods thereof that generate robust nucleic acid transfection in both *in vitro* and *in vivo* systems and exhibit the uprising potential for delivering nucleic acid therapeutics for the treatment of cancer and infectious diseases.

## Description

### CROSS-REFERENCE TO RELATED APPLICATION

The present application claims the benefit of U.S. Provisional Application Serial No. 63/788,315, filed April 14, 2025, which is hereby incorporated by reference herein in its entirety, including any figures, tables, or drawings.

### BACKGROUND OF THE INVENTION

There has been an uprising focus on enhancing the therapeutic efficacy and safety of biological interventions in the field of drug delivery¹⁻⁵. In recent years, mRNA-based therapies have gained widespread attention due to their clinical benefits such as strong treatment outcomes, self-adjuvant properties, and relatively fast clearance from the body⁶⁻⁸. Nonetheless, effective delivery of nucleic acids, for example, mRNA, into target cells (a process also known as transfection) remains a major challenge⁹. Naked mRNAs exhibit instability from RNase-based degradation which justifies the requirement of formulating them with drug carriers, like phase-separating peptides or lipid nanoparticles, to encapsulate and transport the mRNAs to their desired sites¹⁰⁻¹⁵. Conventional mRNA lipid-based carriers may experience some drawbacks, such as premature drug release, lack of stability, low biodegradability, and cellular toxicity¹⁶⁻¹⁹. These limitations reduce the intracellular translation capacity of the mRNAs into the therapeutic proteins^{20, 21}. Hence, the development of novel reagents to effectively deliver mRNA drug payloads is vital for the enhancement of mRNA-based therapies²²⁻²⁶.

Recently, phase-separating peptides have emerged as a suitable drug delivery system that allows excellent intracellular delivery capabilities, good biodegradability, low cellular toxicity, and stable drug release²⁷⁻³⁰. Unique amino acid sequences (spacer and sticker) can form coacervates through liquid-liquid phase separation³¹. Liquid-liquid phase separation enables the generation of dynamic and reversible biomolecular coacervates via the delicate balance between attractive and repulsive forces among biomolecules³². Under physiological conditions, the peptide coacervates provide a droplet-like structure to carry nucleic acids into the cell³³⁻⁵⁵. This technology offers an effective strategy for nucleic acid drug delivery. Phase-separating peptide coacervates exhibit unique characteristics, such as providing high intracellular concentrations of biomolecules, rapid translation of cargo, and protection against cargo degradation³⁶⁻⁴⁰. Together, these benefits reveal the significance of phase-separating peptide-based carriers in revolutionizing the delivery approach for nucleic acids, such as mRNAs and offering an attractive alternative to lipid nanoparticles⁴¹⁻⁴³. Additionally, lipid nanoparticles utilized in vaccines have the side effects of systemic inflammatory response due to the usage of cation lipids. Consequently, preliminary works have been achieved to support this pioneering concept. Recent work by Yang *et al.* reveals the potential for phase-separating peptide formulations to deploy RNAs for efficient intracellular bio-detector applications⁴⁴. Previously, Ries *et al.* demonstrated that mRNA's phase separation could be supported by m⁶A, the most prevalent nucleotide modification in typical mRNAs⁴⁵. Alternatively, Sun *et al.* established phase-separating peptides for transporting CRISPR/Cas9-based gene editors⁴⁶. Together, these examples accentuate the utility of phase-separating peptide-based delivery systems in various nucleic acid-based applications. However, these peptides suffer from the following limitations: (1) most of these reported peptides are lengthy, which increases the risk of immunogenicity in animals; (2) the transfection efficiency cannot be compared with commercially available transfection agents, such as lipofectamine; and (3) transfection or delivery of nucleic acid drugs using peptide coacervates in animals has not been reported. Furthermore, a safe and effective mRNA vaccine formulation is urgently needed for a more effective drug delivery vehicle of mRNA drug payloads into human cells.

### BRIEF SUMMARY OF THE INVENTION

The subject invention pertains to a novel nucleic acid delivery system, also called a transfection system. In one aspect, the subject invention discloses a nucleic acid delivery system comprising a phase-separating peptide coacervate system, where the nucleic acids are encapsulated in the phase-separating peptide coacervates. In embodiments, the amino acid sequence of the phase-separating peptide is WWKssKWW. In certain embodiments, the phase-separating peptide coacervate is adapted for delivering nucleic acids, including RNA and/or DNA, inside a cell. In certain embodiments, the nucleic acid, e.g., mRNA, encodes a vaccine. In certain embodiments, the mRNA encodes a drug. In preferred embodiments, the drug comprises a tumor antigen. In preferred embodiments, the drug comprises a virus antigen.

In another aspect, the subject invention discloses a method for delivering nucleic acids encoding a drug inside one or more cells in a subject in need thereof, comprising: (a) obtaining a phase-separating peptide; (b) generating a phase-separating peptide coacervate comprising the phase-separating peptide; (c) obtaining a nucleic acid, such as an mRNA or DNA encoding a drug and/or a vaccine; (d) mixing the mRNA or DNA encoding the drug and/or the vaccine with the phase-separating peptide coacervate to obtain a droplet that encapsulates the mRNA or DNA; and (e) administering the droplet to the subject to generate an immune response. In certain embodiments, the droplet is administered as part of a composition. In certain embodiments, the composition comprises the droplet and optionally excipients and/or carriers. In certain embodiments, the composition optionally comprises an adjuvant. In preferred embodiment, the immune response produces a remission of a cancer or viral infection, where the mRNA encodes a drug. In preferred embodiment, the immune response produces immunity in the subject, where the mRNA encodes a vaccine. In some embodiments, the subject is a mammal, and the mammal is preferably a human.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.

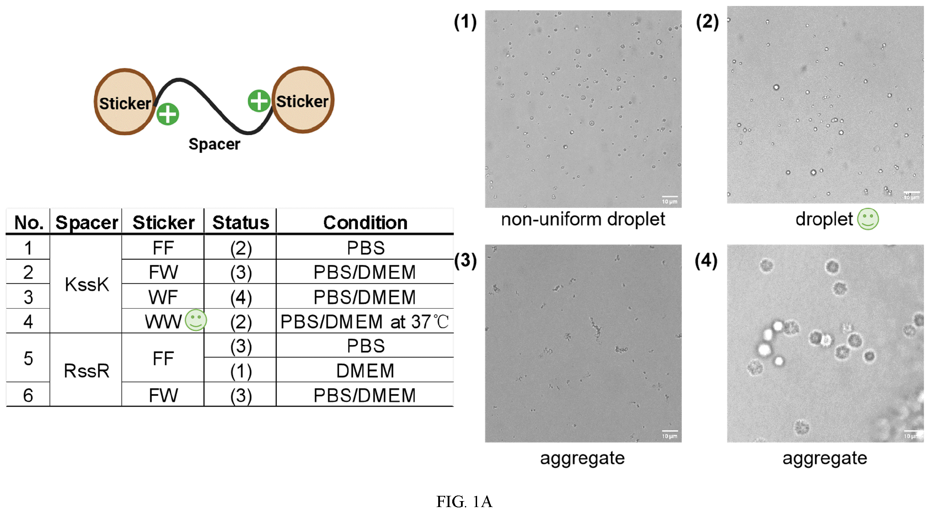

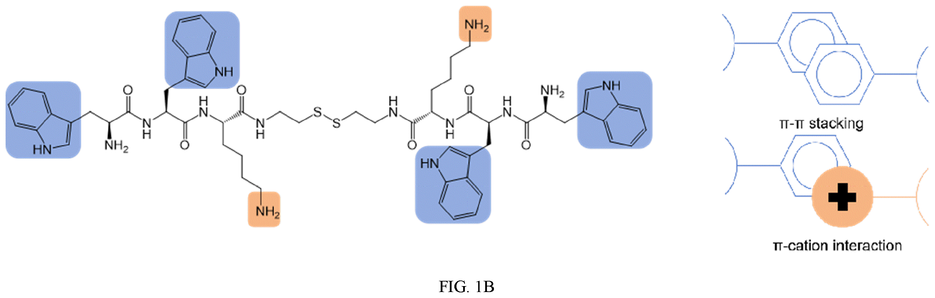

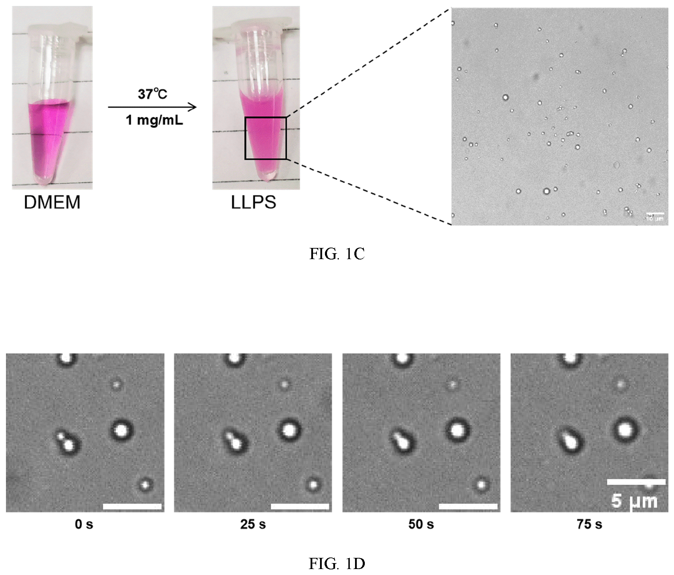

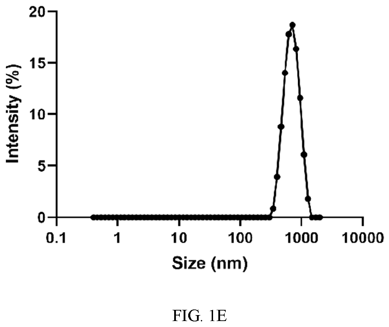

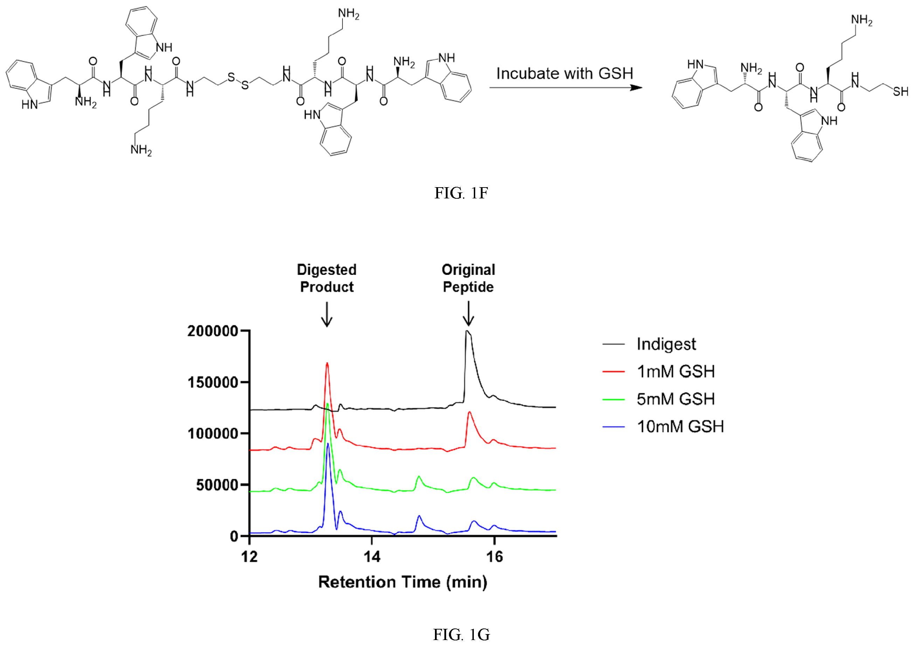

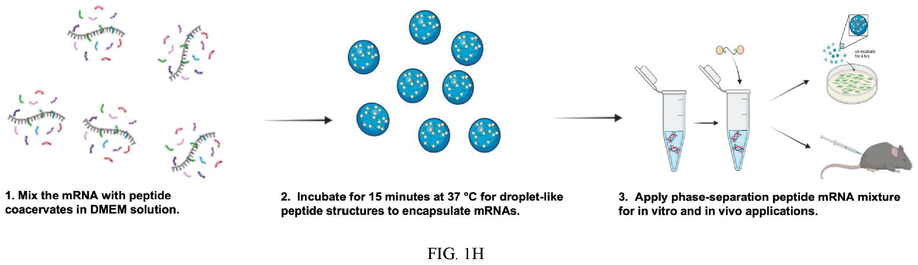

Figures 1A-1H illustrate the selection and synthesis of phase-separating peptide coacervates for nucleic acid delivery. Figure 1A is a schematic illustration of different spacers and stickers combinations for coacervates selection. **Figure 1B** shows bright-field microscopy images of various spacers and stickers coacervates status. The WWKssKWW motif forms droplet-like structures at 37°C in DMEM. **Figure 1C** illustrates the time-lapse dynamic fusion characteristics of WWKssKWW coacervates in DMEM at 37°C. Scale bars, 10 µm. **Figure 1D** illustrates the recruitment of nucleic acids by WWKssKWW coacervates. **Figure 1E** illustrates the size of WWKssKWW coacervates formed in PBS solution. **Figure 1F** illustrates the chemical structure of WWKssKWW before and after GSH addition. **Figure 1G** illustrates the redox-based liquid chromatography analysis of WWKssKWW using GSH. **Figure 1H** illustrates a schematic of simple procedure to prepare phase-separating peptide mRNA formulation for *in vitro* and *in vivo* biological applications.

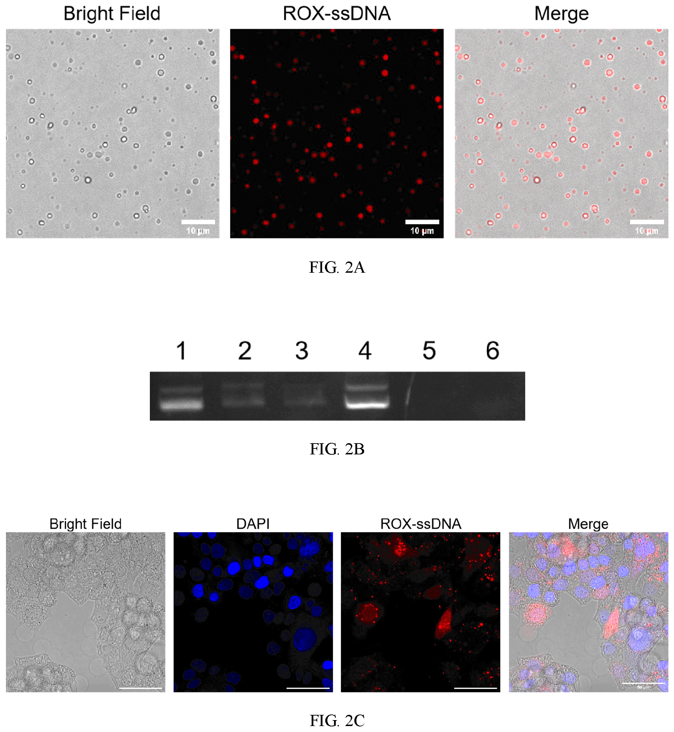

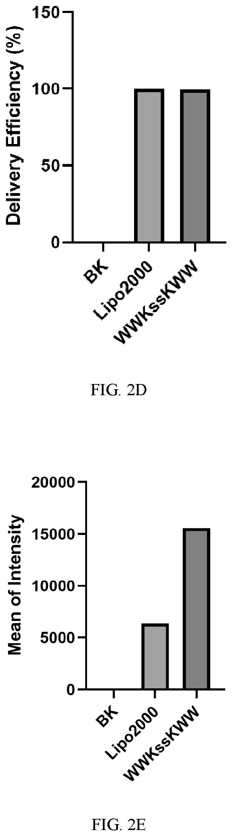

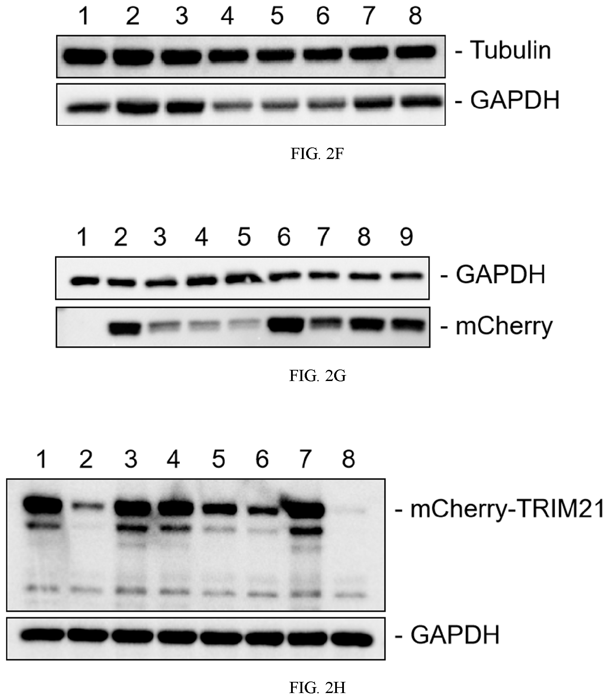

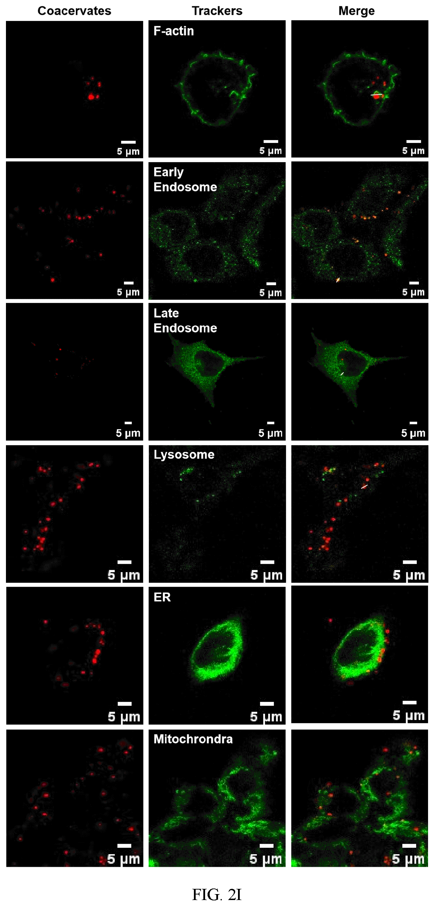

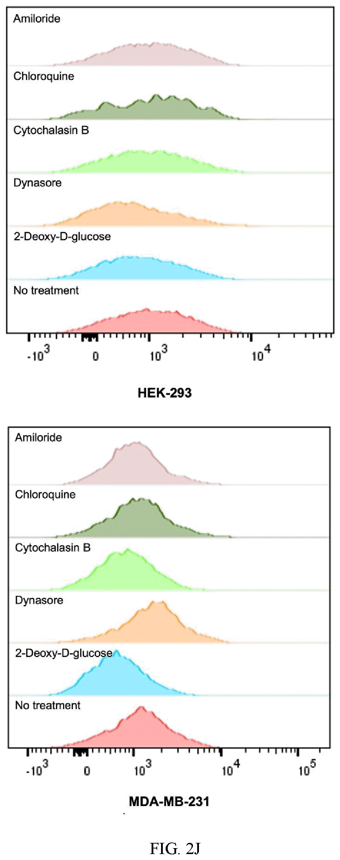

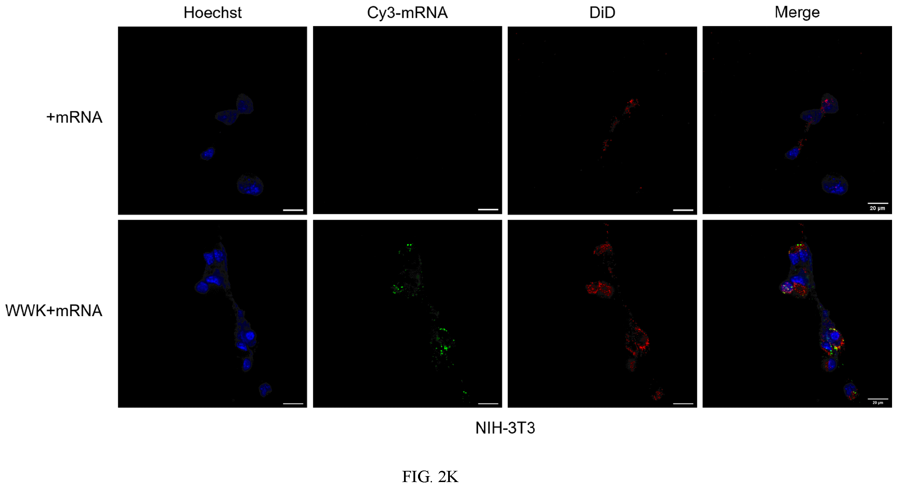

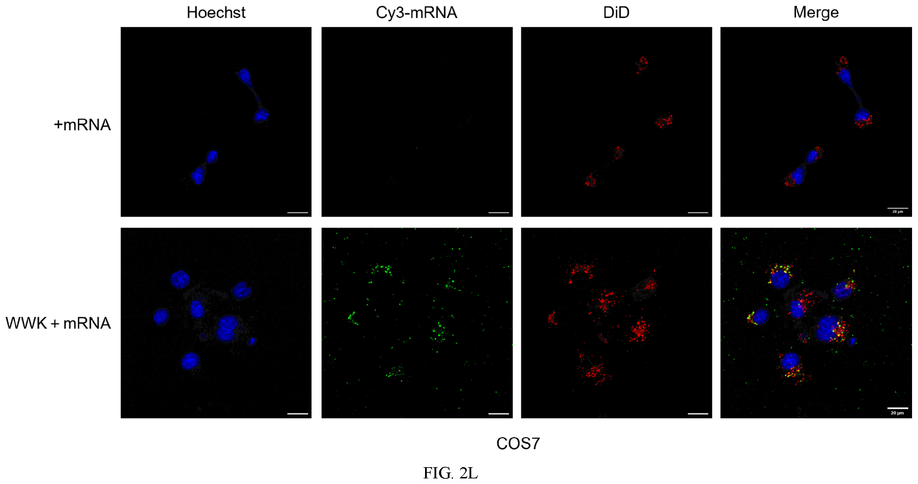

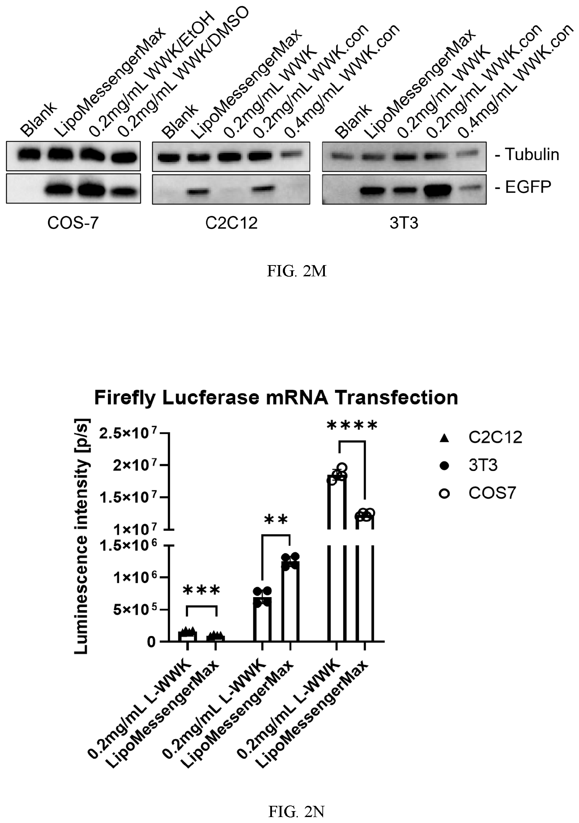

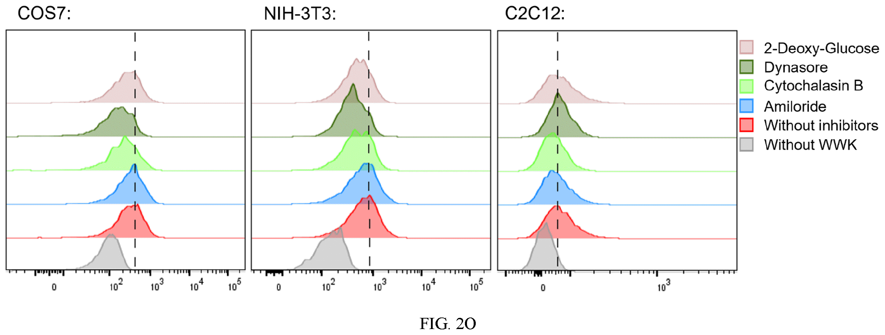

**Figures 2A-2O** **illustrate the recruitment and cargo delivery of nucleic acids by phase-separating peptides.** **Figure 2A** shows that ROX labeled single strand DNA can be recruited by WWKssKWW coacervates. **Figure 2B** shows that plasmids can be recruited by WWKssKWW coacervates. **Figure 2C** shows that some coacervates had already undergone digestion within the cytosol, releasing the ROX-ssDNA into the cytoplasm, and **Figure 2D** shows that the delivery efficiency of WWKssKWW coacervates is comparable to that of the commercial reagent Lipofectamine^{®} 2000. **Figure 2E** shows that the mean fluorescent intensity of cells in the coacervates group is significantly higher than that in the Lipofectamine^{®} 2000 group, and **Figure 2F** shows the WWKssKWW coacervates-based delivery platform for siGAPDH achieves significantly enhanced knockdown efficacy relative to commercial reagents like lipofectamine 3000 and PEI (Lane 1: 0.3 mg/mL peptide and 24h incubation; Lane 2: Lipo3000 and 24h incubation; Lane 3: PEI and 24h incubation; Lane 4: 0.2 mg/mL peptide and 48h incubation; Lane 5: 0.3 mg/mL peptide and 48h incubation; Lane 6: Lipo3000 and 48h incubation; Lane 7: PEI and 48h incubation; and Lane 8: Blank). **Figure 2G** shows that the expression level of mCherry in 0.3 mg/mL WWKssKWW coacervates groups is 1.5 times higher than that in the PEI group in 48 hours (Lane 1: Blank; Lane 2-5: incubate 24h: Lane 2: lipo3000 + 1 µg plasmid; Lane 3: PEI + 1 µg plasmid; Lane 4: 0.3 mg/mL WWKssKWW + 1 µg plasmid; Lane 5: 0.3 mg/mL WWKssKWW+ 1 µg plasmid + 3 mM GSH; Lane 6-9: incubate 48h: Lane 6: lipo3000 + 1 µg plasmid; Lane 7: PEI + 1 µg plasmid; Lane 8: 0.3 mg/mL WWKssKWW+ 1 µg plasmid; and Lane 9: 0.3 mg/mL WWKssKWW+ 1 µg plasmid + 3 mM GSH). **Figure 2H** shows, within just 24 hours, that the expression level of mCherry-TRIM21 in the 0.3 mg/mL WWKssKWW coacervates group is approximately 7.2 times greater than that observed in the lipofectamine^{®} 3000 group. Furthermore, at the 48-hour mark, the expression levels in the coacervates groups remains significantly elevated compared to the lipofectamine^{®} 3000 group (Lane 1: 0.3 mg/mL peptide and 24h incubation; Lane 2: Lipo3000 and 24h incubation; Lane 3: PEI and 24h incubation; Lane 4: 0.2 mg/mL peptide and 48h incubation; Lane 5: 0.3 mg/mL peptide and 48h incubation; Lane 6: Lipo3000 and 48h incubation; Lane 7: PEI and 48h incubation; and Lane 8: Blank). These data show that the coacervates formed by WWKssKWW are general transfection agents for all types of nucleic acids. **Figure 2I** shows the uptake mechanism of WWKssKWW coacervates into HEK-293 cells. Polymerization of F-actin are found around the coacervates on the surface of the cells to help the coacervates entering the cells. During the early stage of condensate entry into cells, the fluorescent signal of the coacervates partially overlaps with the Rab5A signal which is an early endosome marker. After 6 hours, the detection of Rab7A, which is a late endosome marker, and lysosomal signals reveals no overlap between the coacervates' fluorescent signal and either marker, indicating that although the condensates undergo endosomal uptake, they successfully escape from the endosomal system. In addition, the WWKssKWW coacervates does not colocalize with mitochrondria and ER. **Figure 2J** shows that WWKssKWW coacervates partially penetrated cell membrane via the dynamin-mediated endocytosis pathway in HEK-293 cells. These inhibitors targeting various endocytosis pathways, including dynasore (an inhibitor of dynamin-mediated endocytosis), amiloride (an inhibitor of pinocytosis), cytochalasin B (an inhibitor of pinocytosis and phagocytosis), 2-deoxy-D-glucose (an inhibitor of energy dependent endocytosis) and serum starvation (removal of serum to inhibit energy-dependent endocytosis), are utilized to evaluate the uptake mechanisms. The flow cytometry results indicate that the serum starvation group exhibits a reduction of over 50% in coacervates uptake in HEK-293 cells, compared to the positive control group, suggesting that WWKssKWW coacervates are likely to enter the cells via energy-dependent pathways. Dynasore group exhibits a pronounced decline in the uptake of coacervates, confirming that WWKssKWW coacervates partially penetrate cell membrane via the dynamin-mediated endocytosis pathway in HEK-293 cells. The uptake mechanism is completely different in MDA-MB-231 cells. Cytochalasin B significantly inhibits the uptake of WWKssKWW coacervates, indicating that macropinocytosis pathway is the major uptake pathway in MDA-MB-231 cells. In contrast, the dynamin-mediated endocytosis inhibitor dynasore does not show any effect on the coacervates uptake. In sum, the uptake mechanism of WWKssKWW differs with different types of cells. **Figure 2K** shows that the WWKssKWW coacervates recruited the Cy3-labeled mRNA and entered the cell cytosol of NIH-3T3 cells. **Figure 2L** shows that the WWKssKWW coacervates recruited the Cy3-labeled mRNA and entered the cell cytosol of COS7 cells. **Figure 2M** shows that the WWKssKWW coacervates-based delivery platform for EGFP mRNA achieved equivalent protein expression level of EGFP compared to the commercial transfection reagent Lipofectamine MessengerMax in different cell lines. **Figure 2N** shows that the WWKssKWW coacervates-based delivery platform for Firefly Luciferase mRNA achieved significantly enhanced protein expression level of Firefly Luciferase compared to Lipofectamine MessengerMax in C2C12 and COS7 cells, though the performance of the coacervates was comparatively lower in NIH-3T3 cells. **Figure 2O** shows that WWKssKWW coacervates partially penetrated cell membrane via the dynamin-mediated endocytosis pathway in COS7 and NIH-3T3 cells. Meanwhile, in C2C12 cells, WWKssKWW coacervates partially penetrated cell membrane via the macropinocytosis pathway.

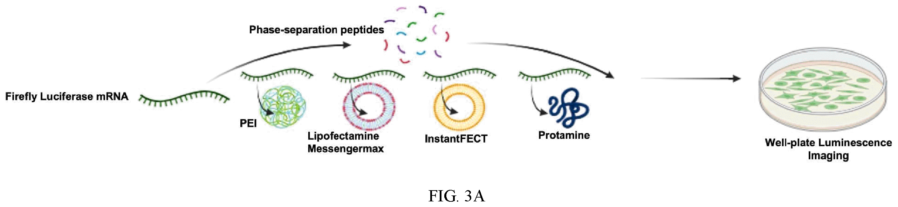

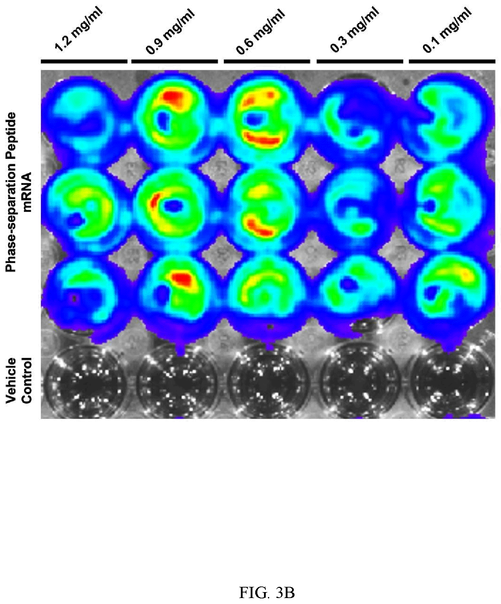

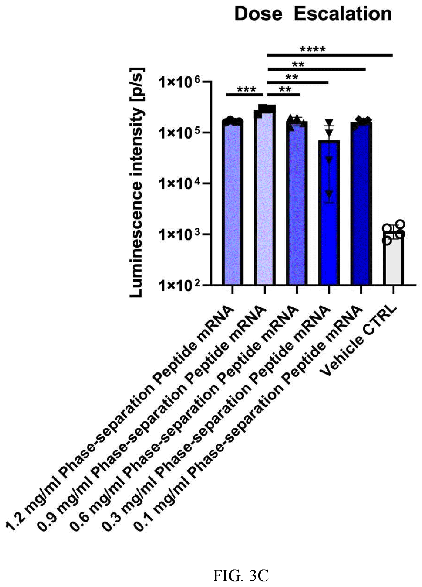

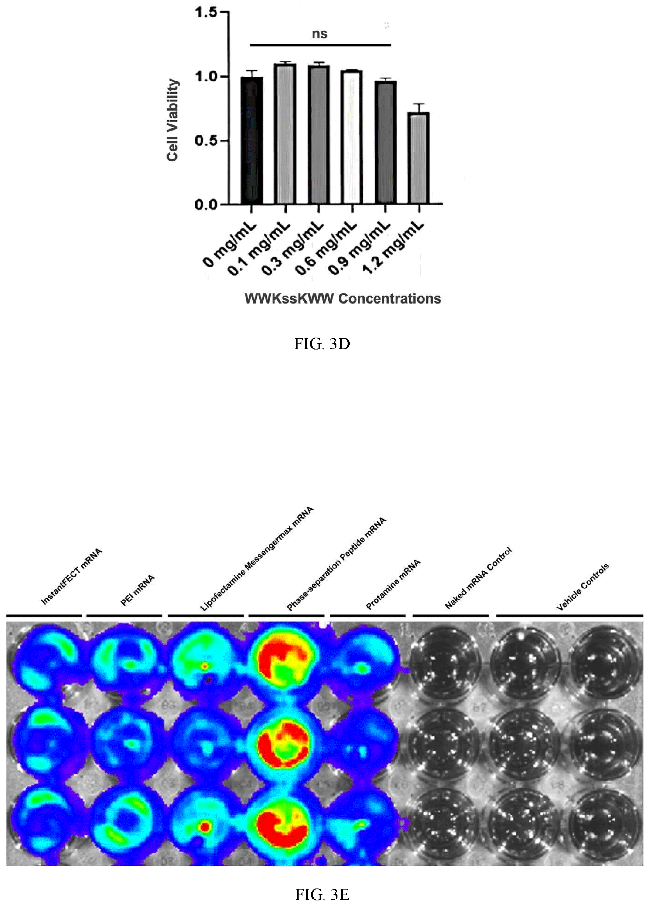

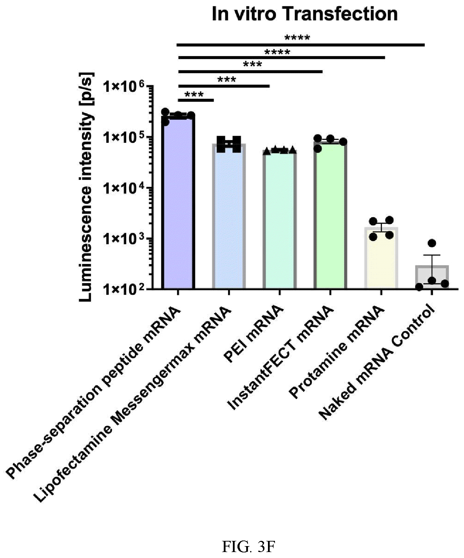

**Figures 3A-3F** **illustrate *in vitro* delivery of luciferase mRNA using phase-separating peptide coacervates.** **Figure 3A** shows a schematic head-to-head comparison of various transfection reagents for mRNA *in vitro* expression. **Figure 3B** shows IVIS well-plate imaging of luciferase mRNA (1µg/well) expression in HEK293 cells by various phase-separating peptide concentrations (0.1 mg/ml, 0.3 mg/ml, 0.6 mg/ml, 0.9 mg/ml, 1.2 mg/ml). **Figure 3C** illustrates quantification of mRNA luminescence from various well groups from **Figure 3B** (n=4). Experiments were repeated at least two times. **Figure 3D** illustrates Cell Viability assay of various phase-separating peptide (WWKssKWW) concentrations for mRNA (1µg/well) delivery. "ns" refers to non-significant. **Figure 3E** shows IVIS well-plate imaging of luciferase mRNA (1µg/well) expression by phase-separating peptide (0.9mg/ml), Lipofectamine^{™} MessengerMAX^{™}, InstantFECT, PEI, protamine, and naked mRNA. **Figure 3F** illustrates quantification of mRNA luminescence from various well groups from **Figure 3E** (n=4). Experiments were repeated at least two times. Unpaired T-test was used for statistical assessment.

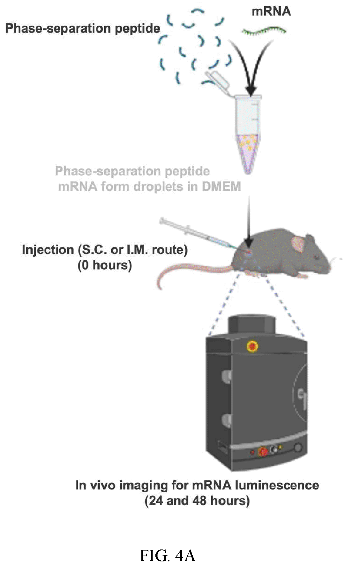

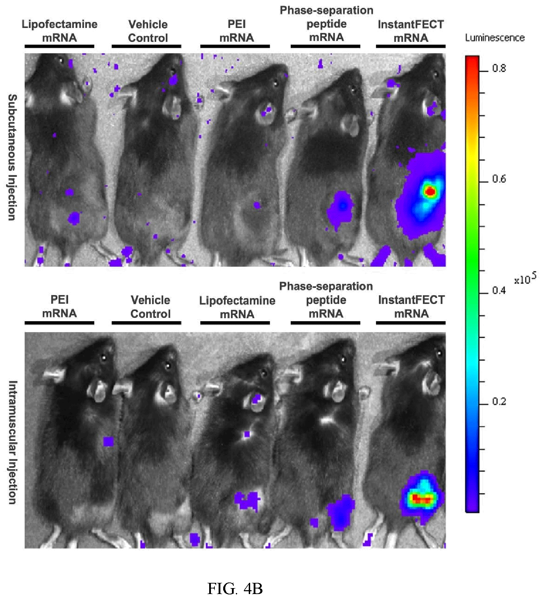

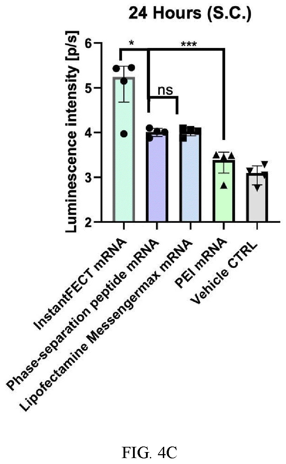

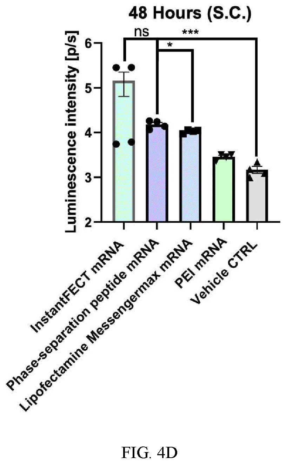

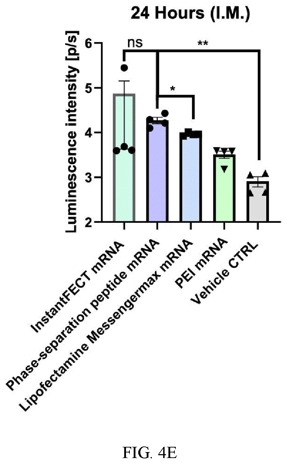

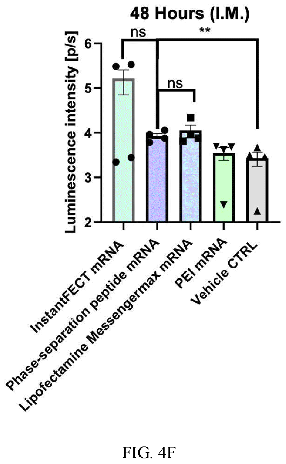

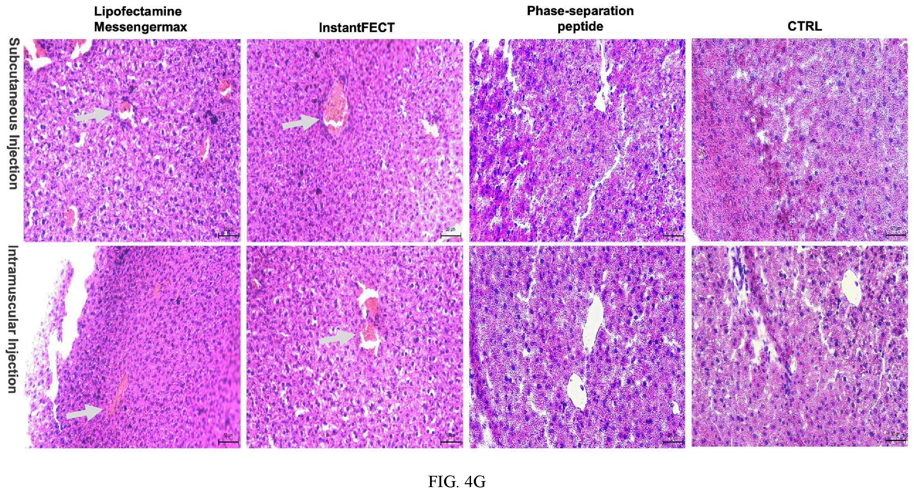

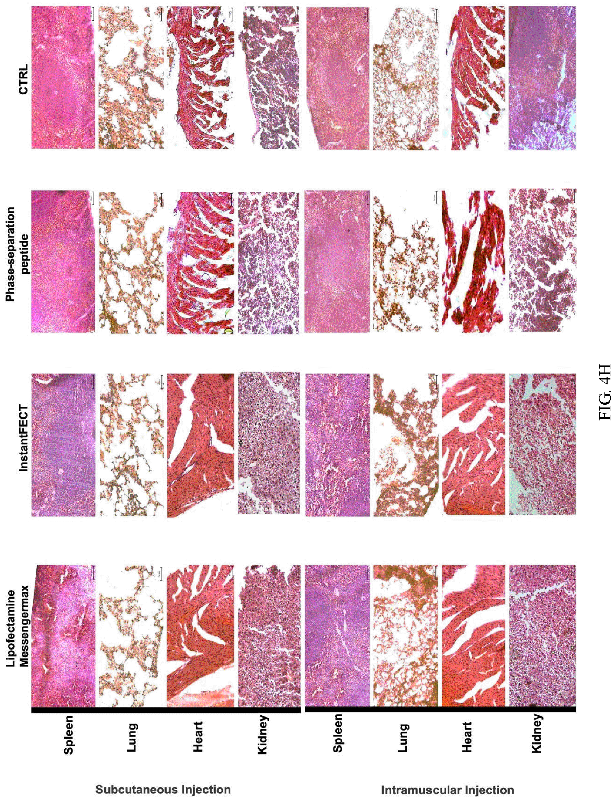

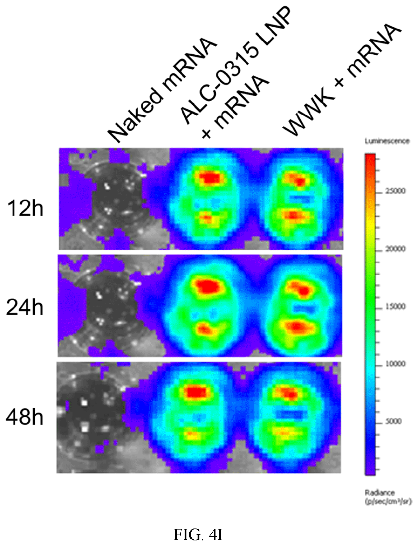

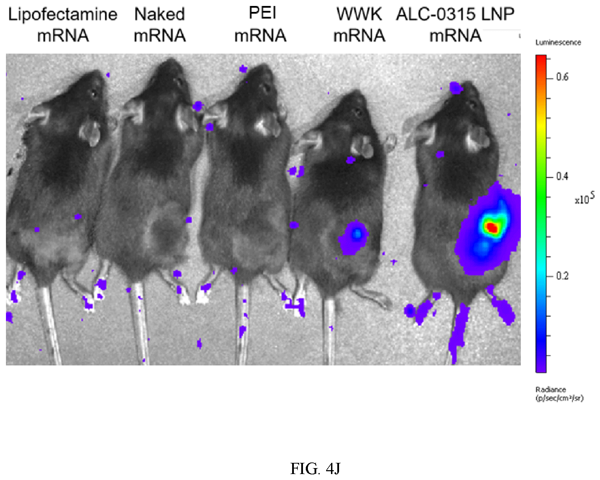

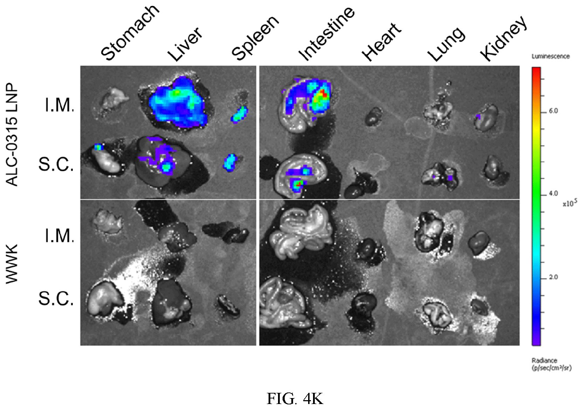

**Figures 4A-4K** **illustrate *in vivo* delivery of luciferase mRNA using phase-separating peptide coacervates.** **Figure 4A** is a schematic illustration of subcutaneous phase-separating peptide coacervates/mRNA administration. **Figure 4B** shows IVIS *in vivo* imaging of mice subcutaneously or intramuscularly injected with various luciferase mRNA (10 µg) formulations. **Figures 4C** and **4D** show quantification of mRNA luminescence from subcutaneously injected mice at 24 hours **(****Figure 4C****)** and 48 hours **(****Figure 4D****)** (n=4/group). Experiments were repeated at least two times. Quantification of mRNA luminescence from intramuscularly injected mice at 24 hours **(****Figure 4E****)** and 48 hours **(****Figure 4F****)** (n=4/group). Unpaired T-test was used for statistical assessment. "****" refers to P values less than 0.0001. "ns" refers to non-significant. **Figure 4G** shows histological liver congestion regions (grey arrows) present 24 hours after injection of various mRNA formulations. No liver congestion was observed in the phase-separating peptide group. Scale bars = 50µm. **Figure 4H** shows histological assessment of various organs (heart, lung, spleen, and kidney) after 24 hours injection of various mRNA formulations. Scale bars = 50µm. **Figure 4I** illustrates that *in vitro* transfection over 48 hours showed similar levels of luciferase mRNA expression (1µg/well) by WWKssKWW coacervates and ALC-0315 based lipid nanoparticles (LNPs) detected by IVIS well-plate software. **Figure 4J** illustrates that after the subcutaneous injection of luciferase mRNA (10µg/mice) packaged by Lipofectamine MessengerMax, PEI, WWKssKWW coacervates and ALC-0315 LNPs, the WWKssKWW coacervate-based delivery platform achieved higher protein expression level compared to lipofectamine but lower than ALC-0315 LNPs. At 24 hours after S.C. and I.M. mRNA administration, the mice were sacrificed, and various organs (stomach, liver, spleen, intestine, heart, lungs and kidney) were harvested and subjected to IVIS detection. **Figure 4K** illustrates that mRNA delivered by WWKssKWW coacervate-based delivery platform only expressed around the injection site, but mRNA delivered by ALC-0315 LNPs would express also in the stomach, lung, liver, spleen, kidney and intestine of the mice.

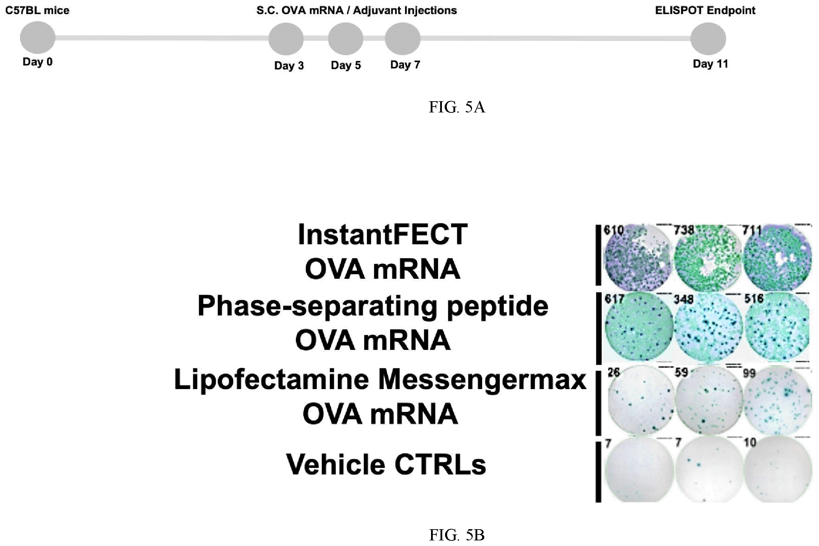

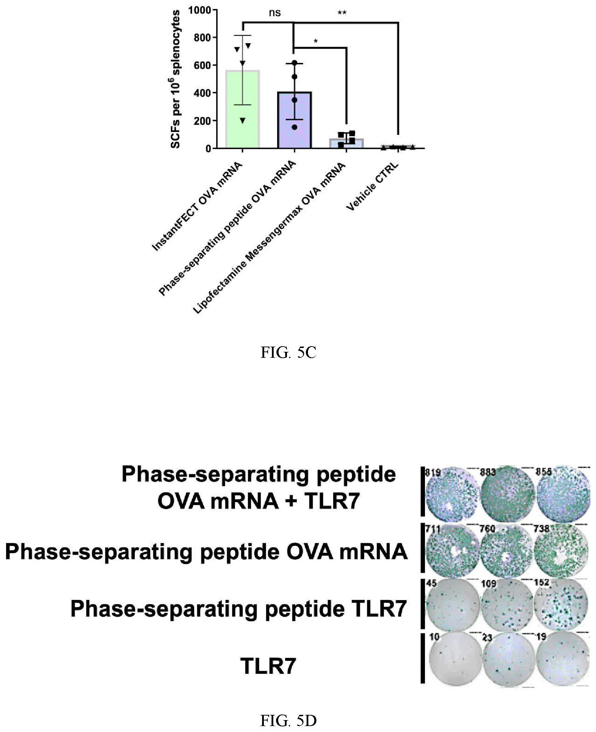

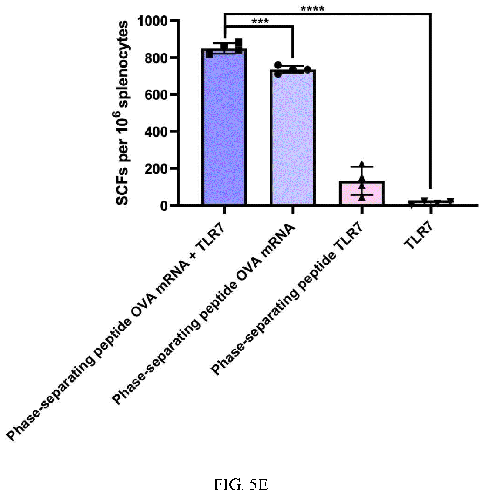

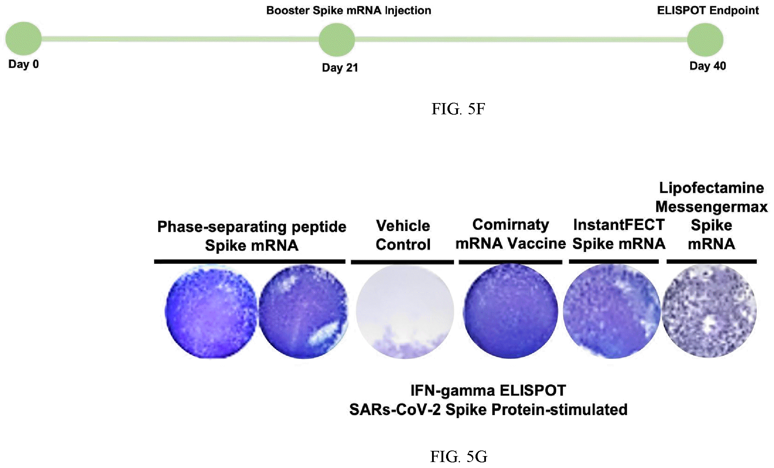

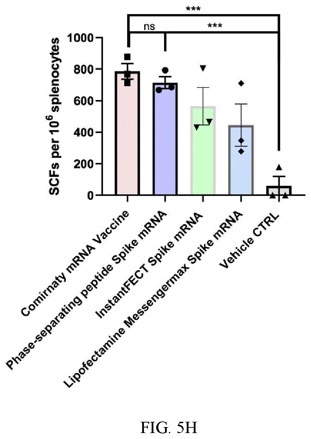

**Figures 5A-5H** **illustrate cellular immunogenicity of antigen mRNAs and TLR7 peptide delivery by phase-separating peptide coacervates.** **Figure 5A** illustrates OVA antigen mRNA (25 µg/mice) subcutaneous injections or TLR7 to mice by phase-separating peptides. **Figure 5B** shows IFN-gamma ELISPOT wells from phase-separating peptide, Lipofectamine^{™} MessengerMAX^{™}, InstantFECT, and vehicle control injected mice. **Figure 5C** illustrates quantification of ELISPOT wells from groups in **Figure 4B** (n=4/group). Experiments were repeated at least two times. **Figure 5D** shows IFN-gamma ELISPOT wells from phase-separating peptide OVA mRNA, phase-separating peptide TLR7, phase-separating peptide combination, and TLR7-alone groups. **Figure 5E** shows quantification of ELISPOT wells from groups in **Figure 4D** (n=4/group). Experiments were repeated at least two times. **Figure 5F** schematically illustrates spike antigen mRNA (15µg/mice) intramuscular injections to mice by phase-separating peptides. **Figure 5G** shows IFN-gamma ELISPOT wells from phase-separating peptide spike mRNA, Comirnaty mRNA vaccine, InstantFECT spike mRNA, Lipofectamine^{™} MessengerMAX^{™} spike mRNA, and phase-separating peptide vehicle control groups. **Figure 5H** illustrates quantification of ELISPOT wells from groups in **Figure 4G** (n=3/group). Unpaired T-test was used for statistical assessment. "****" refers to P values less than 0.0001. "***" refers to P values less than 0.001. "**" refers to P values less than 0.01. "*" refers P values less than 0.05.

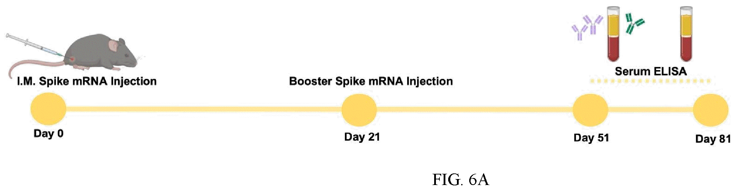

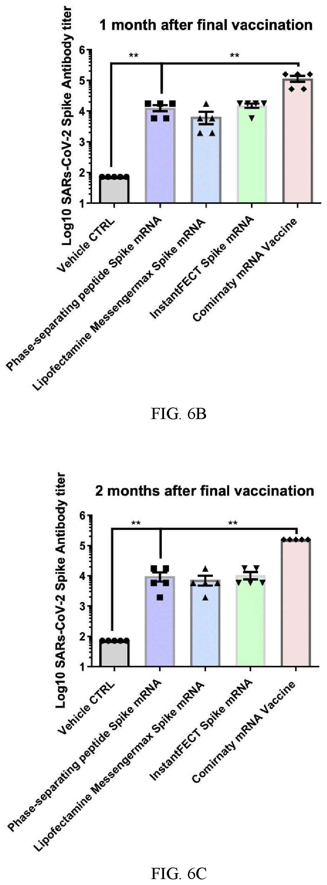

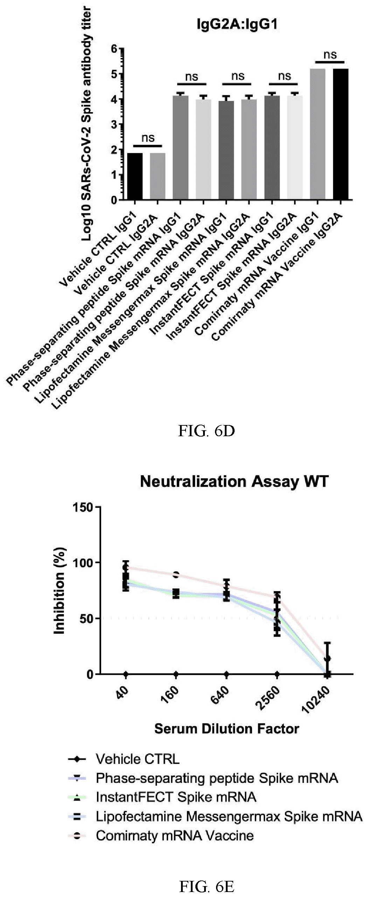

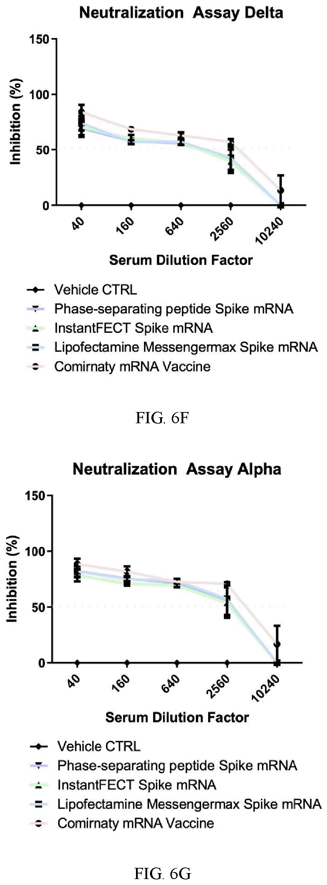

**Figures 6A-6G** **illustrate humoral immunogenicity of SARS-CoV-2 spike mRNA delivery by phase-separating peptide coacervates.** **Figure 6A** schematically illustrates spike antigen mRNA (25 µg/mice) intramuscular injections to mice by phase-separating peptides. **Figure 6B** illustrates SARS-CoV-2 Spike-specific antibody responses at 1 month after final vaccination from phase-separating peptide spike mRNA, Comirnaty mRNA vaccine, InstantFECT spike mRNA, Lipofectamine^{™} MessengerMAX^{™} spike mRNA, and phase-separating peptide vehicle control groups. **Figure 6C** illustrates SARS-CoV-2 Spike-specific antibody responses at 2 months after final vaccination from phase-separating peptide spike mRNA, Comirnaty mRNA vaccine, InstantFECT spike mRNA, Lipofectamine^{™} MessengerMAX^{™} spike mRNA, and phase-separating peptide vehicle control groups. **Figure 6D** illustrates the ratio of IgG1-mediated and IgG2A-mediated humoral immunity from various treatment groups at 2 months. Kolmogorov-Smirnov test was used for statistical analysis. **Figure 6E** illustrates neutralizing antibody titers against WT strain of SARS-CoV-2 from various experimental groups at 2 months after final vaccination. **Figure 6F** illustrates neutralizing antibody titers against Delta strain of SARS-CoV-2 from various experimental groups at 2 months after final vaccination. **Figure 6G** illustrates neutralizing antibody titers against Alpha strain of SARS-CoV-2 from various experimental groups at 2 months after final vaccination. "**" refers to P values less than 0.01. "ns" refers to non-significant.

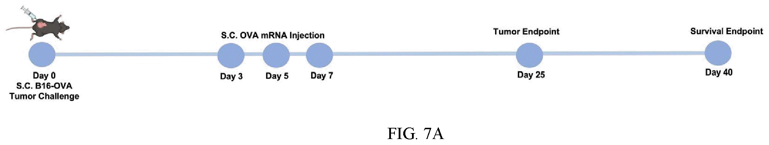

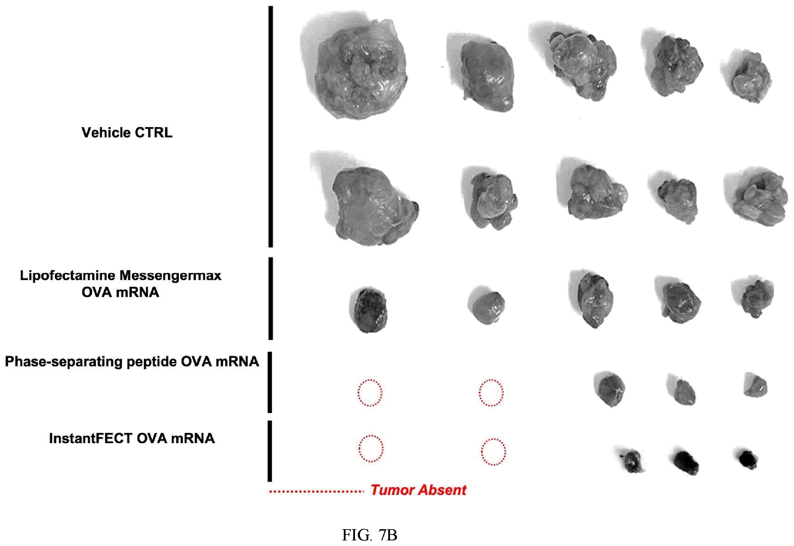

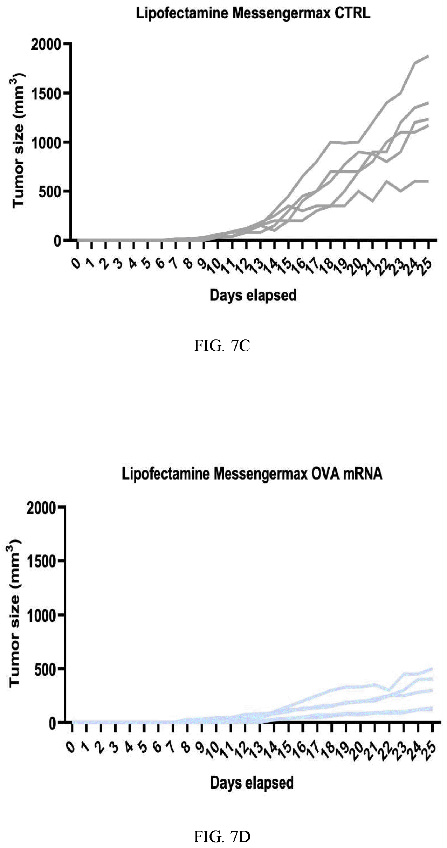

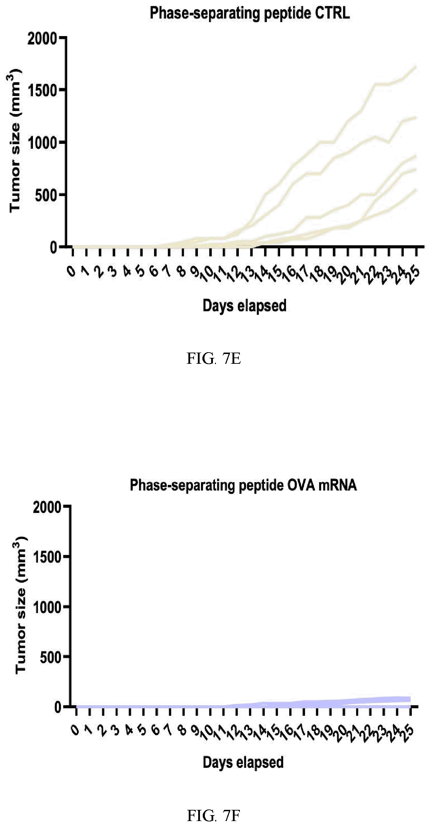

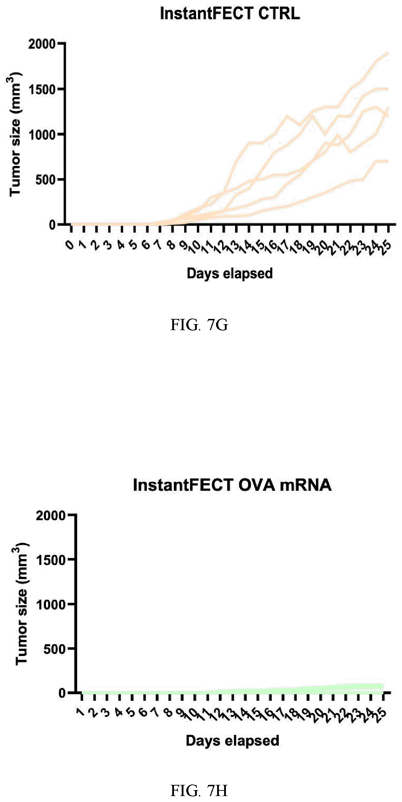

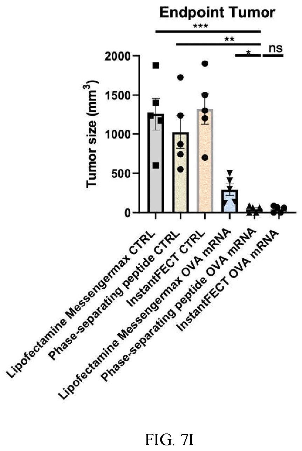

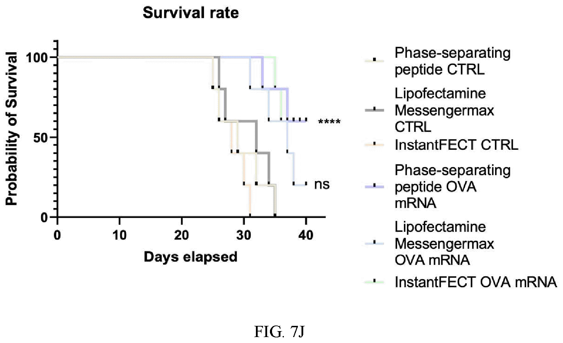

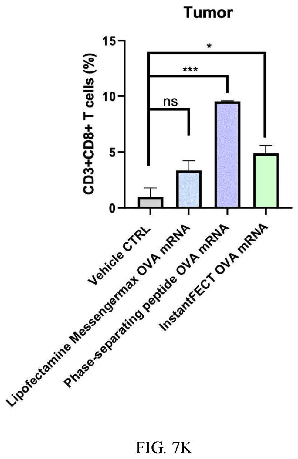

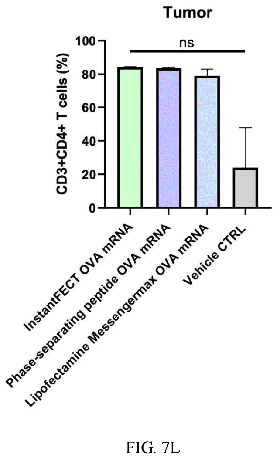

**Figures 7A-7L** **illustrate the efficacy of phase-separating peptide coacervates/OVA mRNA treatment against B16-OVA cancer.** **Figure 7A** schematically illustrates OVA mRNA subcutaneous injections to mice after subcutaneous B16 tumor challenge (2x10⁵ cells/per mice). **Figure 7B** shows a digital image of endpoint tumor sizes from groups in **Figure 5A** (n=5/group). Individual tumor growth of mice from **Figure 5B****.** **Figure 7C** illustrates Lipofectamine^{™} MessengerMAX^{™} CTRL. **Figure 7D** illustrates Lipofectamine^{™} MessengerMAX^{™} OVA mRNA. **Figure 7E** illustrates phase-separating peptide CTRL. **Figure 7F** Phase-separating peptide OVA mRNA. **Figure 7G** illustrates InstantFECT CTRL. **Figure 7H** illustrates InstantFECT OVA mRNA. **Figure 7I** shows quantification of tumor volumes from different groups in **Figure 5B** (n=5/group). **Figure 7J** illustrates survival curve of different groups from **Figure 5B** (n=5/group). Significance was assessed by Mantel-Cox statistical analysis. "***" refers to P values less than 0.001. "**" refers to P values less than 0.01. "*" refers P values less than 0.05. "ns" refers to non-significant. Flow cytometric analysis of tumor infiltrating T-cells (k-l). **Figure 7K** illustrates CD8-positive T cell percentages of different tumor groups. **Figure 7L** illustrates CD4-positive T cell percentages of different tumor groups. Unpaired T-test was used for statistical assessment. "****" refers to P values less than 0.0001. "ns" refers to non-significant.

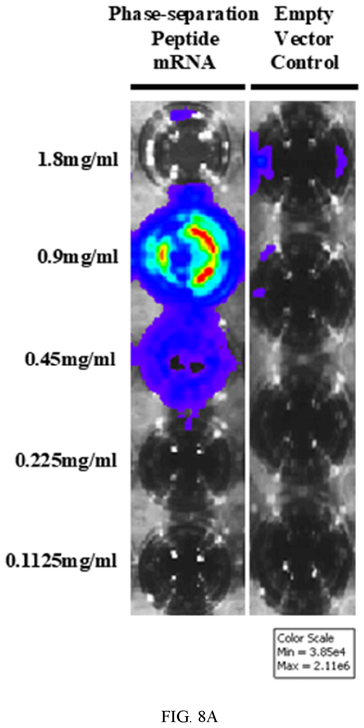

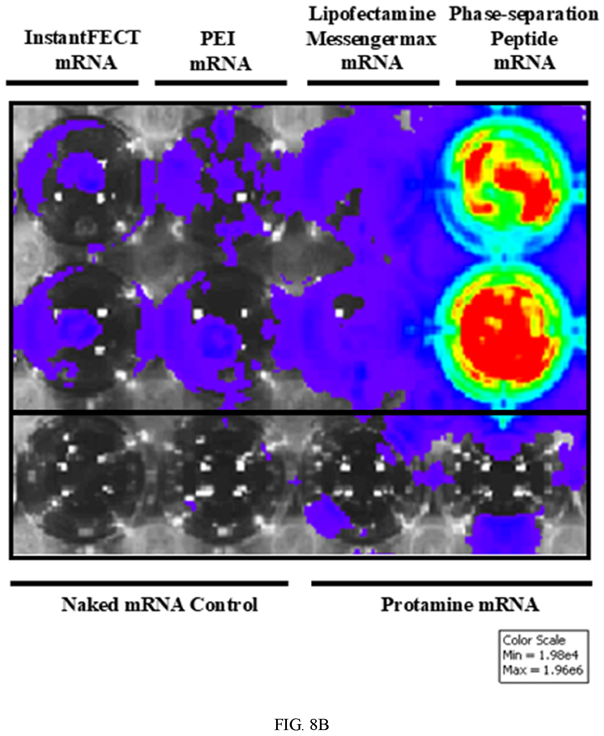

**Figures 8A-8B** show *in vitro* transfection of luciferase mRNA into HEK293 by phase-separating peptide coacervates and various mRNA transfection vectors. **Figure 8A** shows dose-escalation of phase-separating peptides for luciferase mRNA delivery. **Figure 8B** shows head-to-head comparison of optimal dose (0.9 mg/ml) phase-separating peptide mRNA formulation versus InstantFECT mRNA, PEI mRNA, Lipofectamine^{™} MessengerMAX^{™} mRNA, protamine mRNA, and naked mRNA controls.

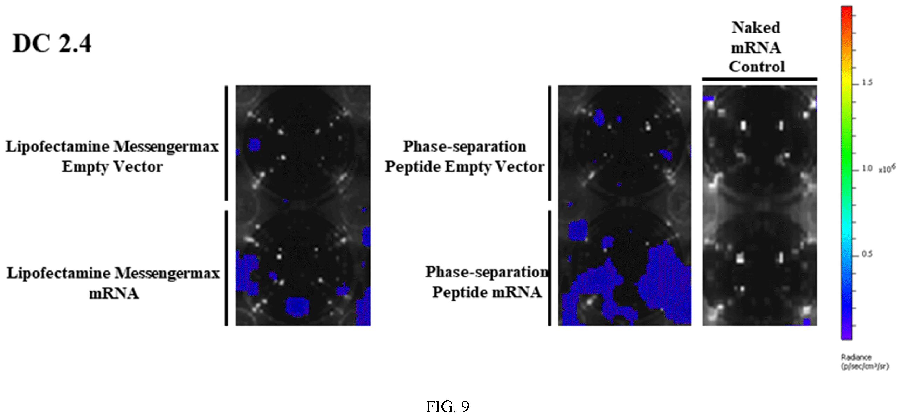

**Figure 9** illustrates *in vitro* transfection of luciferase mRNA into dendritic cells (DC 2.4) by phase-separating peptide coacervates and Lipofectamine^{™} MessengerMAX^{™}.

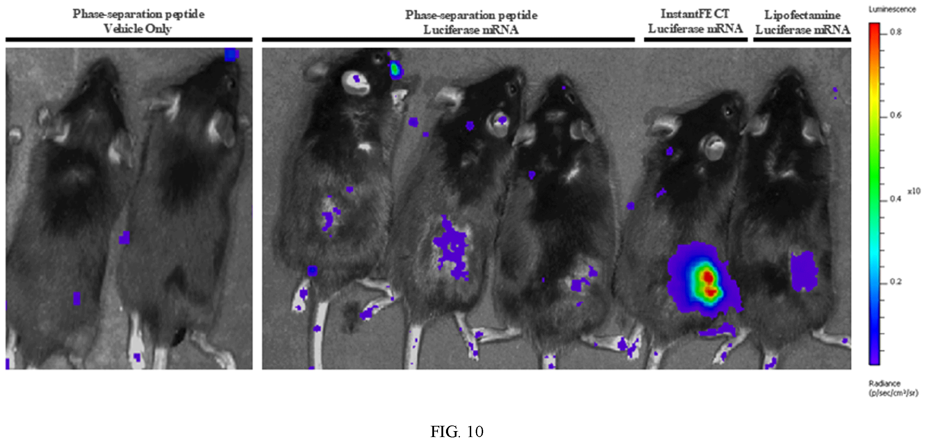

**Figure 10** shows IVIS *in vivo* imaging of mice subcutaneously with various luciferase mRNA (10µg) formulations (phase-separating peptide coacervates/mRNA, phase-separating peptides vehicle only, InstantFECT mRNA, and Lipofectamine.

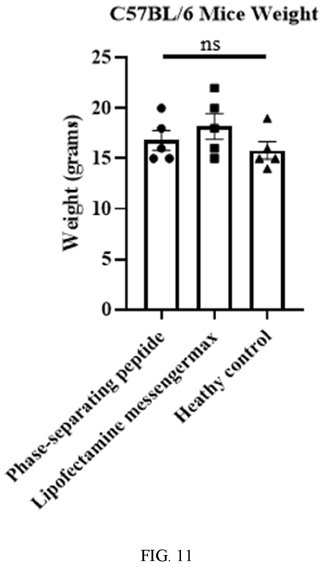

**Figure 11** illustrates C57BL/6 mice weights of 48 hours after injection with phase-separating peptides, Lipofectamine^{™} MessengerMAX^{™} compared to healthy mice controls.

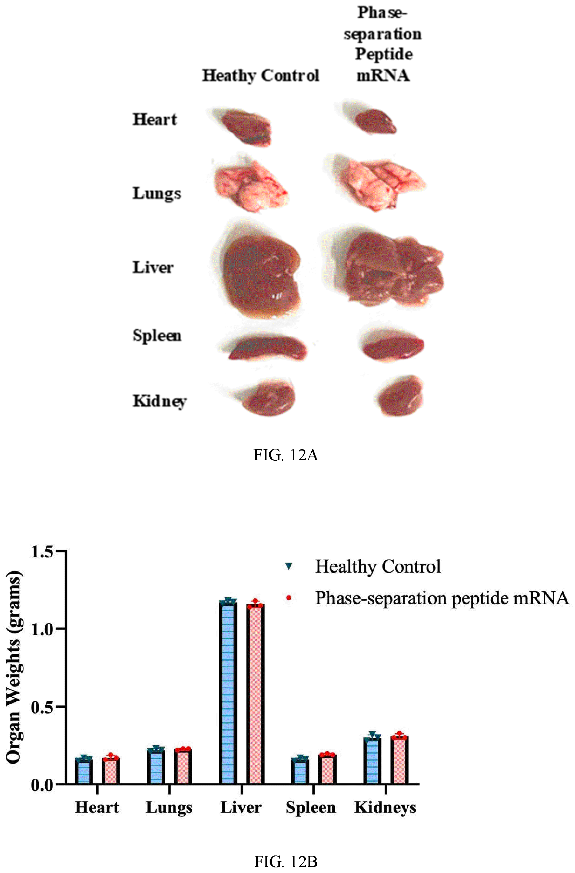

**Figures 12A-12B** illustrate safety of phase-separating peptide coacervates/mRNA compared to non-immunized, healthy controls. 3 days after the final 3 times injection of phase-separating peptide coacervates/mRNA (OVA mRNA), organs were harvested (digital representations) **(****Figure 12A). Figure 12B** illustrates organ weights for comparison with healthy control mice.

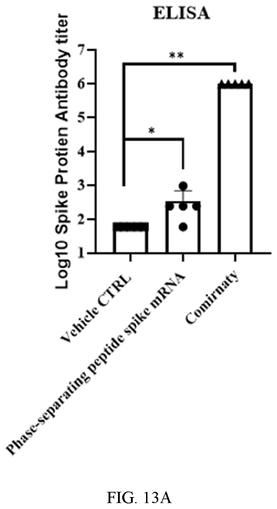

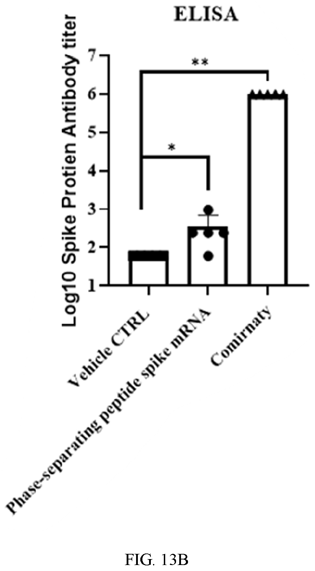

**Figures 13A-13B** illustrate immunogenicity of SARS-CoV-2 Spike mRNA delivered by phase-separating peptides versus Comirnaty. At days 0 and 21 mice were immunized with phase-separating peptide spike mRNA or Comirnaty mRNA vaccine (15µg/mice) intramuscularly. Day 40 anti-spike serum ELISA was performed **(****Figure 13A****).** Day 50 anti-spike serum ELISA was performed **(****Figure 13B****).** Mann-Whitney test was used for statistical analysis. "**" refers to P values less than 0.01. "*" refers P values less than 0.05.

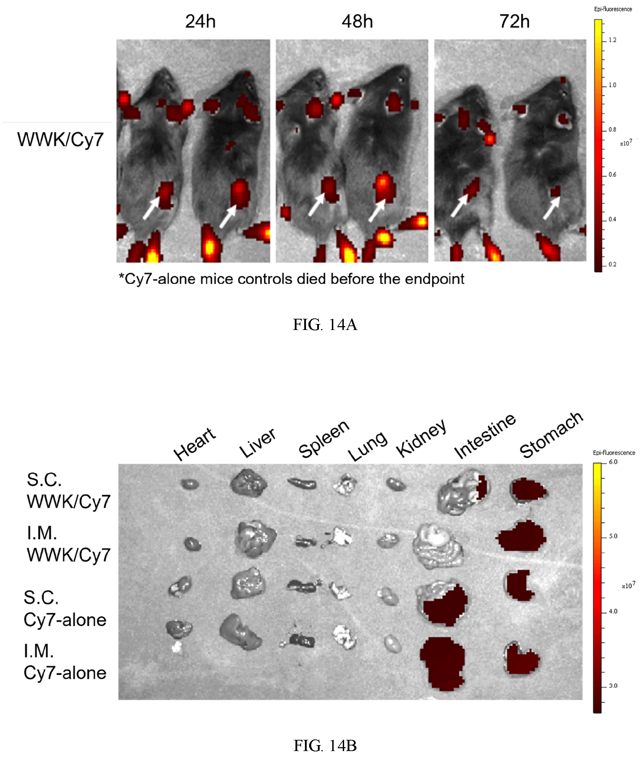

**Figures 14A-14B** illustrate *in vivo* organ distribution monitoring of WWK/Cy7 peptides. IVIS Cy7-scanning after administration of WWKssKWW coacervate-encapsulating Cy7 dyes at 24, 48, and 72 hours (S.C. left, I.M. right) showing localized accumulation of Cy7 at injected areas. White arrows indicate site of injection **(****Figure 14A****).** IVIS Cy7-imaging of mice organs at 72 hours immediately after sacrifice. WWK/Cy7 is mostly localized to injection sites with leakage to the stomach, but most peptides and dyes are digested upon reaching the intestines (possibly by pancreatic enzymes and GST enzymes respectively). Cy7-alone (CTRL) led to wide-spread distribution to the stomach and intestines. Before 72 hours after injection of Cy7-alone (I.M. or S.C.), Cy7-alone mice died possibly due to cytotoxicity **(****Figure 14B****).**

### DETAILED DISCLOSURE OF THE INVENTION

### Selected Definitions

As used herein, the singular forms "a", "an" and "the" are intended to include the plural forms as well, unless the context clearly indicates otherwise. Furthermore, to the extent that the terms "including", "includes", "having", "has", "with", or variants thereof are used in either the detailed description and/or the claims, such terms are intended to be inclusive in a manner similar to the term "comprising". The transitional terms/phrases (and any grammatical variations thereof) "comprising", "comprises", "comprise", "consisting essentially of", "consists essentially of", "consisting" and "consists" can be used interchangeably.

The phrases "consisting essentially of" or "consists essentially of" indicate that the claim encompasses embodiments containing the specified materials or steps and those that do not materially affect the basic and novel characteristic(s) of the claim.

The term "about" means within an acceptable error range for the particular value as determined by one of ordinary skill in the art, which depends in part on how the value is measured, *i.e.*, the limitations of the measurement system. In the context of compositions containing amounts of ingredients where the terms "about" are used, these compositions contain the stated amount of the ingredient with a variation (error range) of 0-10% around the value (X ± 10%). In other contexts, the term "about" is providing a variation (error range) of 0-10% around a given value (X ± 10%). As is apparent, this variation represents a range that is up to 10% above or below a given value, for example, X ± 1%, X ± 2%, X ± 3%, X ± 4%, X ± 5%, X ± 6%, X ± 7%, X ± 8%, X ± 9%, or X ± 10%.

In the present disclosure, ranges are stated in shorthand to avoid having to set out at length and describe each and every value within the range. Any appropriate value within the range can be selected, where appropriate, as the upper value, lower value, or the terminus of the range. For example, a range of 0.1-1.0 represents the terminal values of 0.1 and 1.0, as well as the intermediate values of 0.2, 0.3, 0.4, 0.5, 0.6, 0.7, 0.8, 0.9, and all intermediate ranges encompassed within 0.1-1.0, such as 0.2-0.5, 0.2-0.8, 0.7-1.0, etc. Values having at least two significant digits within a range are envisioned, for example, a range of 5-10 indicates all the values between 5.0 and 10.0 as well as between 5.00 and 10.00 including the terminal values. When ranges are used herein, combinations and subcombinations of ranges (*e.g*., subranges within the disclosed range) and specific embodiments therein are explicitly included.

The recitation of a listing of chemical groups in any definition of a variable herein includes definitions of that variable as any single group or combination of listed groups. The recitation of an embodiment for a variable or aspect herein includes that embodiment as any single embodiment or in combination with any other embodiments or portions thereof.

Any compositions or methods provided herein can be combined with one or more of any of the other compositions and methods provided herein.

Other features and advantages of the invention will be apparent from the following description of the preferred embodiments thereof, and from the claims. All references cited herein are hereby incorporated by reference.

As used herein, the terms "therapeutically-effective amount," "therapeutically-effective dose," "effective amount," and "effective dose" are used to refer to an amount or dose of a compound or composition that, when administered to a subject, is capable of treating, preventing, or improving a condition, disease, or disorder in a subject. In other words, when administered to a subject, the amount is "therapeutically effective." The actual amount will vary depending on a number of factors including, but not limited to, the particular condition, disease, or disorder being treated, prevented, or improved; the severity of the condition; the weight, height, age, and health of the patient; and the route of administration.

The term "pharmaceutically acceptable carrier" refers to an excipient that is a carrier or vehicle, such as a suspension aid, solubilizing aid, or aerosolization aid. Remington: The Science and Practice of Pharmacy, The University of the Sciences in Philadelphia, Editor, Lippincott, Williams, & Wilkins, Philadelphia, Pa., 21st Edition (2005), incorporated herein by reference, describes additional compositions and formulations suitable for pharmaceutical delivery of one or more therapeutic compositions and/or pharmaceutical agents. Pharmaceutically acceptable carriers can contain one or more physiologically acceptable compound(s) that act, for example, to stabilize the composition or to increase or decrease the absorption of the active agent(s). Physiologically acceptable compounds can include, for example, carbohydrates, such as glucose, sucrose, or dextrans, antioxidants, such as ascorbic acid or glutathione, chelating agents, low molecular weight proteins, protection and uptake enhancers such as lipids, compositions that reduce the clearance or hydrolysis of the active agents, or excipients or other stabilizers and/or buffers.

The term "excipient," as used in this disclosure, is an additive that is used in combination with the antigens. An excipient can be used, for example, to dilute an active agent, such as an antigen, and/or to modify properties of a pharmaceutical composition. Examples of excipients include, but are not limited to, water, magnesium stearate, stearic acid, vegetable stearin, sucrose, lactose, starches, hydroxypropyl cellulose, hydoxypropyl methylcellulose, xylitol, sorbitol, maltitol, gelatin, polyethyleneglycol (PEG), phosphate buffered saline (PBS), carboxy methyl cellulose, vitamin A, vitamin E, vitamin C, retinyl palmitate, selenium, cysteine, methionine, citric acid, methyl paraben, propyl paraben, sugar, silica, talc, magnesium carbonate, sodium starch glycolate, tartrazine, aspartame, benzalkonium chloride, sesame oil, propyl gallate, sodium metabisulphite, lanolin, polyvinylpyrrolidone (PVP), tocopheryl polyethylene glycol 1000 succinate (also known as vitamin E TPGS, or TPGS), dipalmitoyl phosphatidyl choline (DPPC), trehalose, sodium bicarbonate, glycine, sodium citrate, lactose, saline, phosphate buffered saline or organic buffers including but not limited to Tris(hydroxymethyl)aminomethane, and metal chelating agents. Metal chelating agents include, but are not limited to, organic compounds such as the amino acids glutamic acid and histidine, organic diacids such as malate, and polypeptides such as phytochelatin, biomolecules such as pyochelin, pyoverdine, enterobactin and Dopa, and synthetic chelates such as ethylenediaminetetraacetic acid (EDTA).

The term "antigen" refers to a compound, composition, or substance that can stimulate the production of antibodies or a T-cell response in an animal, including compositions that are injected or absorbed into an animal. An antigen reacts with the products of specific humoral or cellular immunity. For example, SARS-CoV-2 antigen refers to any portion or fragment of a SARS-CoV-2 polypeptide coded by an mRNA that is recognized by any anti-SARS-CoV-2 antibody or antibody against any portion of SARS-CoV-2. In some cases, the portion or fragment may be a peptide with an attached moiety, such as, but not limited to, a sugar moiety, a phosphate moiety, or a lipid moiety. Alternatively, the portion or fragment may be a peptide without any attached non-peptide moieties.

As used herein, the term "subject" refers to an animal, needing or desiring delivery of the benefits provided by a therapeutic compound. As used herein, the term "animal" may be, for example, humans, pigs, horses, goats, cats, dogs, apes, chimpanzees, orangutans, guinea pigs, hamsters, cows, or sheep. These benefits can include, but are not limited to, the treatment of a health condition, disease, or disorder; prevention of a health condition, disease or disorder; immune health; enhancement of the function of an organ, tissue, or system in the body. The preferred subject in the context of this invention is a human. The subject can be of any age or stage of development, including infant, toddler, adolescent, teenager, adult, or senior.

As used herein, the term "treatment" refers to eradicating; reducing; ameliorating; abatement; remission; diminishing of symptoms or delaying the onset of symptoms; slowing in the rate of degeneration or decline; making the final point of degeneration less debilitating; and/or improving a subject's physical or mental well-being or reversing a sign or symptom of a health condition, disease or disorder to any extent, and includes, but does not require, a complete cure of the condition, disease, or disorder. Treating can be curing, improving, or partially ameliorating a disorder. "Treatment" can also include improving or enhancing a condition or characteristic, for example, bringing the function of a particular system in the body to a heightened state of health or homeostasis.

As used herein, "preventing" a health condition, disease, or disorder refers to avoiding, delaying, forestalling, or minimizing the onset of a particular sign or symptom of the condition, disease, or disorder. Prevention can, but is not required, to be absolute or complete; meaning, the sign or symptom may still develop at a later time. Prevention can include reducing the severity of the onset of such a condition, disease, or disorder, and/or inhibiting the progression of the condition, disease, or disorder to a more severe condition, disease, or disorder.

As used herein, "subject" refers to an animal, such as a mammal, for example a human. The methods described herein can be useful in both humans and non-human animals. In some embodiments, the subject is a mammal (such as an animal model of disease), and in some embodiments, the subject is a human. The terms "subject" and "patient" can be used interchangeably. The animal may be for example, humans, pigs, horses, goats, cats, mice, rats, dogs, apes, fish, chimpanzees, orangutans, guinea pigs, hamsters, cows, sheep, birds, chickens, as well as any other vertebrate or invertebrate.

As used herein, an "isolated" or "purified" compound is substantially free of other compounds. In certain embodiments, purified compounds are at least 60% by weight (dry weight) of the compound of interest. Preferably, the preparation is at least 75%, more preferably at least 90%, and most preferably at least 99%, by weight of the compound of interest. For example, a purified compound is one that is at least 90%, 91%, 92%, 93%, 94%, 95%, 98%, 99%, or 100% (w/w) of the desired compound by weight. Purity is measured by any appropriate standard method, for example, by column chromatography, thin layer chromatography, or high-performance liquid chromatography (HPLC) analysis.

The term "nucleic acid" or "polynucleotide" refers to deoxyribonucleic acids (DNA) or ribonucleic acids (RNA) and polymers thereof in either single- or double-stranded form, both natural and non-natural. The term is not limited by length (e.g., number of monomers) of the polymer. A nucleic acid may be single-stranded or double-stranded and will generally contain 5'-3' phosphodiester bonds, although in some cases, nucleotide analogs may have other linkages. Nucleic acids may include naturally occurring bases (adenosine, guanidine, cytosine, uracil and thymidine) as well as non-natural bases. The term "non-natural nucleotide" or "modified nucleotide" refers to a nucleotide that contains a modified nitrogenous base, sugar or phosphate group, or that incorporates a non-natural moiety in its structure. Examples of non-natural nucleotides include dideoxynucleotides, biotinylated, aminated, deaminated, alkylated, benzylated and fluorophor-labeled nucleotides. Unless specifically limited, the term encompasses nucleic acids containing known analogs of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (*e.g*., degenerate codon substitutions), alleles, orthologs, single nucleotide polymorphisms (SNPs), and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)). The term nucleic acid is used interchangeably with gene, cDNA, and mRNA encoded by a gene. Additionally, the terms "mRNA" and "mRNAs" are used interchangeably.

The term "vaccine" refers to a preparation of immunogenic material capable of stimulating an immune response, administered for the prevention, amelioration, or treatment of disease, such as an infectious disease. The immunogenic material may include, for example, antigenic proteins, peptides derived from a nucleic acid sequence (e.g., DNA or mRNA). Vaccines may elicit prophylactic (preventative) and/or therapeutic responses. Methods of administration vary according to the vaccine, but may include inoculation, ingestion, inhalation or other forms of administration as discussed herein. Inoculations can be delivered by any of a number of routes, including parenteral, such as intravenous, subcutaneous, intramuscular, intranasal, oral, vaginal or rectal. Vaccines may be administered with an adjuvant to boost the immune response.

The term "immune response" refers to a response of a cell of the immune system, such as a B-cell, T-cell, macrophage or polymorphonucleocyte, to a stimulus such as an antigen. An immune response can include any cell of the body involved in a host defense response, including for example, an epithelial cell that secretes an interferon or a cytokine. An immune response includes, but is not limited to, an innate immune response or inflammation. As used herein, a protective immune response refers to an immune response that protects a subject from infection (prevents infection or prevents the development of disease associated with infection) or a cancer.

The term "gene" means the segment of DNA involved in producing a polypeptide chain; it includes regions preceding and following the coding region (leader and trailer) involved in the transcription/translation of the gene product and the regulation of the transcription/translation, as well as intervening sequences (introns) between individual coding segments (exons).

The term s "phase-separating tripeptide dimer" and "phase-separating peptide" can be used interchangeably.

In certain embodiments, treatments are administered to a subject that "significantly reduce or abolish the expression of a gene or functioning of a protein encoded by the gene." This phrase refers to a reduction of gene expression in an amount of at least (or at least about) 30% as compared to a non-treated subject. Thus, the treated subjects exhibit significantly reduced or abolished expression of a gene exhibit a reduction in gene expression or expression of an active protein that can range from about 30% to about 99.99% about 40% to about 99.99%, about 50% to about 99.99%, about 60% to about 99.99%, about 70% to about 99.99%, about 80% to about 99.99%, about 90% to about 99.99% or are devoid of expression (expression is abolished) of the gene or an active protein encoded by the gene.

By "reduces" is meant a negative alteration of at least 1%, 5%, 10%, 25%, 50%, 75%, or 100%.

By "increases" is meant as a positive alteration of at least 1%, 5%, 10%, 25%, 50%, 75%, or 100%.
As used herein, the terms "determining," "measuring," and "assessing," and "assaying" are used interchangeably and include both quantitative and qualitative determinations.
As used herein, the term "droplet" is intended to include its plural form as well.

### Compositions

The subject invention pertains to a nucleic acid delivery system and methods thereof, utilizing a novel phase-separating peptide. Based on the needs to effectively deliver a nucleic acid vaccine , we develop short peptides (10 residues or less) that form phase-separated coacervates, which can deliver the nucleic acids into cells, respond to the cellular environment, and release the nucleic acids into the cytosol, showing an efficiency equivalent or higher than commercial transfection agents such as lipofectamine *in vitro* and in animals. The capability of the peptides to efficiently deliver mRNA, or other nucleic acids, makes their application ideal for mRNA vaccine delivery for the treatment of cancer and infectious diseases. Phase-separating peptide forms coacervates, which comprise micron-scale carriers capable of effective membrane permeation, enabling the encapsulation and cytosolic delivery of nucleic acids, including mRNAs.

In one aspect, the subject invention comprises an mRNA vaccine or drug delivery system, comprising a phase-separating peptide coacervate, where mRNAs are encapsulated in the phase-separating peptide coacervate adapted for delivery inside a cell. In certain embodiments, the phase-separating peptide has amino acid sequence WWKssKWW. In preferred embodiments, the phase-separating peptide forms WWKssKWW coacervates. In preferred embodiments, the mRNA delivered by the WWKssKWW coacervate-based delivery system is only expressed around the injection site. In certain embodiments, the mRNA delivered by the WWKssKWW coacervate-based delivery system does not spread to organs, such as, for example, stomach, lung, liver, spleen, kidney and/or intestine.

In preferred embodiments, the WWKssKWW coacervates accumulate at injected areas. In certain embodiments small amounts of the WWKssKWW coacervates may leak to the stomach.

In a second aspect, the subject invention comprises a method for delivering a nucleic acid, such as an mRNA vaccine or drug inside a cell, comprising obtaining a phase-separating peptide, where the phase-separating peptide has amino acid sequence WWKssKWW, where the phase-separating peptide generates a coacervate, where nucleic acids are mixed with the phase separation peptide coacervate to obtain a droplet that encapsulates the nucleic acid, where the droplet is administered to a subject to generate an immune response.

In a third aspect, the subject invention comprises a composition, where the composition comprises an mRNA vaccine delivery system, comprising a phase-separating peptide coacervate, where the phase-separating peptide has amino acid sequence WWKssKWW, and optionally an adjuvant. In certain embodiments, the composition optionally comprises excipients (e.g., sodium chloride, RNase-free water), adjuvants (*e.g.*, TLR7) and/or carriers (*e.g.*, WWKssKWW phase-separating peptide). In some examples, the composition may comprise excipients: (1) sodium chloride solution (0.5-1%w/v, 10% volume of total vaccine), (2) RNase-free water (to dilute mRNA solutions), and carriers (3) carriers: phase-separating peptide WWKssKWW (for delivering the mRNA drug which is the active ingredient). In certain embodiments, the composition is delivered inside a cell.

In certain examples, the cell is in the form of a multitude of cells, or a tissue, or an organ, or a system, or an organism.

In certain embodiments, the WWKssKWW coacervates can partially penetrate the cell membrane via the dynamin-mediated endocytosis pathway in cells including, but not limited to, HEK-293, COS7, and NIH-3T3. In further embodiments, the WWKssKWW coacervates can partially penetrate the cell membrane via the macropinocytosis pathway in cells including, but not limited to, C2C12 and MDA-MB-231. In preferred embodiments, the WWKssKWW coacervates can enter the cell cytosol of a cell. In certain embodiments, the uptake mechanism of WWKssKWW coacervates differs with different types of cells. In some embodiments, coacervate droplets comprise the phase-separating tripeptide dimer of amino acid sequence WWKssKWW, wherein nucleic acids are encapsulated in the droplets, wherein the nucleic acids encode one or more vaccines or drugs. In further embodiment, wherein the phase-separating tripeptide dimer comprises unnatural amino acids.

In certain embodiments, the mRNAs delivered by the subject invention include, but are not limited to, one or more vaccines. In certain embodiments, the mRNAs delivered by the subject invention include, but are not limited to, a vaccine that triggers immunity, or an immune response, against a pathogen. In certain embodiments, the mRNAs delivered by the subject invention include, but are not limited to, one or more types of cancer, including different mRNAs encoding antigens that trigger immunity against the one or more types of cancer. In preferred embodiment, the mRNA delivery system of the subject invention does not include or utilize nanoparticles and/or liposomes.

In certain embodiments, the subject invention discloses phase-separating peptide-based mRNA formulations that generate robust protein expression in both *in vitro* and *in vivo* systems. Several experiments have been performed to illustrate the head-to-head comparison of phase-separating peptides with other conventional mRNA delivery agents. The phase-separating peptide luciferase mRNA formulation produces significantly higher *in vitro* luminescence than the Lipofectamine^{™} MessengerMAX^{™} -based mRNA, InstantFECT cationic liposome-based mRNA, PEI-based mRNA, protamine-based mRNA, silica nanoparticles-based mRNA, and the naked mRNA control. Likewise, sufficient *in vivo* expression is established after the subcutaneous or intramuscular injection of the phase-separating peptide mRNA formulations into C57BL/6 mice.

In certain embodiments, the phase-separating peptide-based formulations delivers mRNA therapeutics for the treatment of cancer and other infectious diseases. In certain embodiments, the delivery of various antigen mRNAs (OVA tumor antigen and SARS-CoV-2 spike antigen) and TLR 7 adjuvants by the peptide coacervates generates strong cellular interferon-gamma responses and humoral responses. In preferred embodiments, in preclinical cancer model, the phase-separating peptide OVA mRNA treatment provides strong anti-tumor efficacy and substantially promotes tumor-free survival. These responses are comparable to those of the InstantFECT-delivered OVA mRNA treatment group and are more significant than the lipofectamine-delivered OVA mRNA treatment group.

In some embodiments of the invention, the method comprises administration of multiple doses of the compositions of the subject invention. The method may comprise administration of therapeutically effective doses of a composition comprising the compound or composition thereof of the subject invention as described herein once a week, once a month, once a quarter, twice a year, once a year, or a lower frequency. Moreover, treatment of a subject with a therapeutically effective amount of the compositions of the invention can include a single treatment or can include a series of treatments. It will also be appreciated that the effective dosage of a compound or composition thereof used for treatment may increase or decrease over the course of a particular treatment. Changes in dosage may result and become apparent from the results of diagnostic assays determine the presence (or absence) or dental caries and/or carious lesions, which are known in the art. Specifically, the identification of dental caries and/or carious lesions includes, for example, intraoral radiographs.

### MATERIALS AND METHODS

### Phase-separating peptide synthesis

The phase separating peptide was synthesized with Boc protected amino acids in liquid phase. The final product was purified by reverse phase HPLC and validated MOLDI-TOF.
i. N-(tert-Butoxycarbonyl)-N'-(9-fluorenylmethyloxycarbonyl)-L-lysine (Boc-Lys(Fmoc)-OH) (1452 mg, 3.1 mmol), HBTU (1114 mg, 2.94 mmol) and HOBt (398 mg, 2.94 mmol) were dissolved in DMF (6 mL) in a round-bottom flask and the mixture was stirred with a magnetic stirrer. N,NDiisopropylethylamine (DIPEA) (2100 µL, 12.4 mmol), and cystamine dihydrochloride (CDC) (316 mg, 1.4 mmol) were added with an interval of 1 minute respectively and the reaction mixture was stirred for 24 h at room temperature. The reaction mixture was poured into 60 mL of water. White precipitate was collected by filtration and washed with water. The crude product was dried in a desiccator.
ii. The product from the first step (Boc-K(Fmoc)ssK(Fmoc)-Boc) was dissolved in DCM (3 mL) in a round bottom flask. To deprotect the intermediate product, trifluoroacetic acid (TFA) (3 mL) was added and the reaction mixture was stirred for 3 h. The solvent was evaporated on a rotary evaporator, yielding oily residue. Diethyl ether (45 mL) was added to the flask and the content was gently stirred. White precipitate was separated by centrifugation. The crude product was obtained after freeze drying.
iii. Boc-Trp-OH (682 mg, 2.24 mmol), HBTU (804 mg, 2.12 mmol) and HOBt (286 mg, 2.12 mmol) were dissolved in DMF (4.5 mL) in a round-bottom flask and the mixture was stirred with a magnetic stirrer. DIPEA (1560 µL, 9 mmol), and product from the second step (K(Fmoc)ss(Fmoc)K, 853 mg, 1 mmol) was added with an interval of 1 minute respectively and the reaction mixture was stirred for 24 h at room temperature. The reaction mixture was poured into 50 mL of water. The crude product was dried in a desiccator.
iv. The product from the third step (Boc-WK(Fmoc)ssK(Fmoc)W-Boc) was dissolved in DCM (3 mL) in a round bottom flask. TFA (3 mL) was added for deprotection and the reaction mixture was stirred for 3 h. The solvent was evaporated on a rotary evaporator, yielding oily residue. Diethyl ether (45 mL) was added to the flask and the content was gently stirred. A white precipitate formed and was separated by centrifugation. The crude product was obtained after freeze drying.
v. Boc-Trp-OH (247 mg, 1.81 mmol), HBTU (652 mg, 1.72 mmol) and HOBt (232 mg, 1.72 mmol) were dissolved in DMF (3 mL) in a round-bottom flask and the mixture was stirred with a magnetic stirrer. DIPEA (1264 µL, 7.29 mmol), and product from the fourth step (WK(Fmoc)ss(Fmoc)KW, 991 mg, 0.81 mmol) was added with an interval of 1 minute respectively and the reaction mixture was stirred for 24 h at room temperature. The reaction mixture was poured into 50 mL of water. The crude product was dried in a desiccator.
vi. The product from the fifth step (Boc-WWK(Fmoc)ssK(Fmoc)WW-Boc) was dissolved in DCM (1.5 mL) in a round bottom flask. TFA (1.5 mL) was added for deprotection and the reaction mixture was stirred for 3 h. The solvent was evaporated on a rotary evaporator, yielding oily residue. Diethyl ether (45 mL) was added to the flask and the content was gently stirred. A white precipitate formed and was separated by centrifugation. The crude product was obtained after freeze drying.
vii. The product from the sixth step (WWK(Fmoc)ssK(Fmoc)WW) was dissolved in DCM (1.5 mL) in a round bottom flask. Diethylamine (1.5 mL) was added for deprotection and the reaction mixture was stirred for 4.5 h. The solvent was evaporated on a rotary evaporator, yielding oily residue. Diethyl ether (45 mL) was added to the flask and the content was gently stirred. A white precipitate formed and was separated by centrifugation. The final product was obtained after freeze drying, separated by LC and characterized by MALDI mass spectrometry. *m*/*z* calc. for C₆₀H₇₆N₁₄O₆S₂ [M+H]⁺ 1153.55, C₆₀H₇₆N₁₄O₆S₂ [M+Na]⁺ 1175.54, C₆₀H₇₆N₁₄O₆S₂ [M+K]⁺ 1191.65, found 1154.320, 1176.313 and 1192.301.

### Coacervation status characterization

WWKssKWW peptides were dissolved in DMSO (100 mg/mL, stock solution), and then 2 µL stock solution was mixed with 98 µL aqueous solution gently and incubate at 37°C to allow the coacervates to form. The solution gradually became white and milky, indicating the formation of coacervates. Coacervates were visualized under a confocal microscope. WWKssKWW coacervates droplets were observed to fuse together under the confocal microscope.

### Redox-based liquid chromatography analysis

1 µL WWKssKWW stock solution was mixed with 99 µL PBS solution gently and incubate at 37°C for 15 minutes. Glutathione (GSH) was added to the solution at final concentration of 0 mM, 1 mM, 5mM and 10mM. The mixture was incubated at 37°C overnight. All groups were injected to HPLC separately to detect whether the WWKssKWW peptide react with GSH completely. The HPLC program is 5% acetonitrile with 0.1% TFA/95% water with 0.1% TFA for 5 minutes; the concentration of acetonitrile gradiently increase to 95% in 25 minutes; 95% acetonitrile with 0.1% TFA/5% water with 0.1% TFA for 5 minutes. The column is a C18 reverse phase column.

### Cell viability analysis

Cell viability was detected by LDH cytotoxicity assay kit (Beyotime). HEK-293 cells were plated in 96-well cell culture plate at 60% confluency and cultured overnight in 100 µL DMEM supplemented with 10% FBS. Peptide solutions were administered in triplicate wells at designated concentrations (0, 0.1, 0.3, 0.6, 0.9, 1.2 mg/mL). Following 4 h exposure (37°C/5% CO₂), the treatment medium was removed and replaced with fresh medium. After 24 hours, one set of the serial dilutions is lysed and used to determine the maximum LDH Release. Add 10 µL of Lysis Solution to the Maximum LDH Release dilution series, then mix by gentle tapping. The second set of serial dilutions is used to determine the spontaneous LDH release. A dd 10 µL of medium to the spontaneous LDH Release dilution series of triplicate wells containing cells, then mix by gentle tapping. Incubate the plate for 30 minutes in the incubator. Aspirate 50 µL of culture supernatant from each well, and use the remaining culture supernatant as the detection object. Add 50 µL of Working solution to each sample well, then mix by gentle tapping. Incubate the plate at room temperature for 30 minutes protected from light. Add 50 µL of Stop Solution to each sample well, and then mix by gentle tapping. Measure the absorbance at 490 nm using a microplate reader.

### mRNA synthesis

Tobacco mosaic virus (TMV) 5' and 3' untranslated regions (UTR) cloned in pUC57 plasmid was modified by inserting poly-adenine (A) tail (130 bases in length) behind the 3' UTR. The gene of interest was then inserted into the plasmid backbone. *In vitro* transcription was performed using a T7-HiScribe mRNA synthesis kit (NEB). Uridine bases (UTP) were substituted with N-methyl pseudouridine triphosphate (TriLink BioTechnologies). The mRNA products were purified by the lithium chloride precipitation method. 5'-capping was performed using the vaccinia-virus-capping system (NEB). The final mRNA products were reconstituted in RNase-free water.

### In vitro imaging

Luciferase mRNAs (1 µg/well) were mixed and briefly vortexed with Lipofectamine^{™} MessengerMAX^{™} (2.5 µl/well, according to manufacturer's instructions), PEI, InstantFECT cationic liposome, protamine, silica nanoparticles or phase-separating peptide (WWKssKWW, 0.9 mg/ml). Dosage of the mRNA transfection reagents used in this study were adopted from our previous work⁵. For phase-separating peptide mRNA complexation, the mixture was incubated at 37°C in DMEM for 15 minutes prior to transfection. Human embryonic kidney 293 cells (HEK293 cells) were seeded in a 48-well plate (approximately 2.5 × 10⁴ HEK293 cells/ well) and different mRNA formulations were added dropwise into respective wells. Cells were incubated for 24 hours (37 °C in 5% CO₂ atmosphere). After incubation, luciferin substrate (2 µl (30 mg/ml), Gold Biotechnology) was added to the respective wells and plates were imaged by IVIS luminescence imager (Perkin Elmer IVIS spectrum 2020) to detect luciferase signals under well-plate settings.

### In vivo imaging

Luciferase mRNAs (10 µg/mice) were prepared into phase-separating peptide (WWKssKWW, 0.9 mg/ml), PEI, InstantFECT Cationic liposome, or Lipofectamine^{™} formulations (6 µl/mice, according to manufacturer's instructions) before being subcutaneously injected into the hind flank of mice. Dosage of the mRNA transfection reagents used in this study were adopted from our previous work⁵. 24 hours after injection, C57BL/6 mice anesthetized with ketamine and xylazine. Next, luciferin substrate (100 µl (30 mg/ml), Gold Biotechnology) was injected intraperitoneally. After 15 minutes, all mice were place into IVIS imager (IVIS SPECTRUM) for detecting luciferase signals. Region of interest (ROI) signals were quantified on the IVIS software to determine luminescence intensities.

### Animal Experiments

Six-week-old C57BL/6 mice were provided by the Centre of Comparative Medicine (CCMR) at the University of Hong Kong. All animal experiments were approved by the Committee on the Use of Live Animals in Teaching & Research (CULATR), the University of Hong Kong. The murine-derived B16-OVA cell-line (Sigma-Aldrich, SCC420, source information can be provided by supplier upon request) was cultured under mycoplasma negative conditions in Dulbecco's modified Eagle medium (DMEM) with 10% fetal bovine serum (FBS). A total of 2 x10⁵ cells in 100 µL were subcutaneously injected to the flank female C57BL/6 mice using a Hamilton syringe and size 25S gauge bevel tip needle at day 0. Phase-separating peptide (0.9 mg/ml) OVA mRNA (20 µg), Lipofectamine^{™} MessengerMAX^{™} OVA mRNA (20 µg), and vehicle controls were subcutaneously administered at days 3, 5, and 7. At the tumor endpoint (day 25), mice were sacrificed, and primary and metastatic tumors were surgically harvested for tumor size analysis using electronic calipers. Spleens were also surgically harvested and processed for further analysis at day 11 by resuspending strained splenocytes in an FBS-containing RPMI medium, adding red blood cell lysis buffer, and washing in filtered PBS. For survival studies, tumor-bearing mice were closely monitored up to the day 40 endpoint and euthanized according to the humane endpoint guidelines and judged based on the veterinarian's observation for signs and symptoms of pain and distress to minimize harm to tumor-bearing animals. The percentage of weight loss data allowed is 20% of the original body weight for the humane endpoint criteria of all animal tumor experiments. The animal experiments in this study were approved by the Committee on the Use of Live Animals in Teaching & Research, the University of Hong Kong (#CULATR 23-034).

### IFN-γ ELISpot assay

On day 11, mice were sacrificed and splenocytes were harvested for IFN-γ ELISpot analysis. Briefly, 100 µl of splenocytes were incubated on the IFN-γ ELISpot plate, which was preactivated for 30 minutes using 200 µl FBS-containing DMEM media. Subsequently, 5 µg of the peptide (OVA and the positive inducer) were added and cells were incubated for 24 hours at 37 °C in 5% CO₂. Cells were washed by washing buffer and incubated with the secondary biotinylated antibody for 1 hour at room temperature. Finally, the cells were incubated with the biotin substrate, followed by washing and drying to generate visible spots in positive wells. ELISPOT plates were scanned and analyzed by ImmunoSpot software to count the respective wells.

### ELISA

At 1 month and 2 months after the final vaccination, blood obtained from tail vein was centrifuged at 3000 rpm for 30 min. All mice serum was stored (-80 °C) for further characterization. A 96-well ELISA plate was coated with 10 µg/mL of antigen (spike protein) diluted in coating medium. 100 µL of the coating solution was added to each well and kept overnight at 4°C. After 12 h, the solution was discarded, and the ELISA plate was blocked with blocking buffer (5% milk in tris-buffered saline combined with tween 20 [TBST]) for 2 h at room temperature (RT). The wells were then washed with TBST six times. Next, serum collected above was serially diluted in milk-TBST solution at the following ratios: 1:24, 1:72, 1:216, 1:648, 1:1944, 1:5832, 1:17496, 1:52488. The diluted serum was added to the wells and incubated for 1 h at 37°. The plate was washed and then incubated with mouse IgG, mouse IgG1, or mouse IgG2a secondary antibody diluted in milk-TBST solution at a ratio of 1:3000. After 1 h incubation at 37°C, the plate was washed. Next, 100 µL of 3,3',5,5'-Tetramethylbenzidine (TMB substrate) was added to each well and incubated at 37 °C for 30 min. Finally, the reaction was stopped using H₂SO₄ (50 µL/well) and the absorbance collectively was read at 450 nm by a Varioskan Flash spectral scanning multimode reader (Thermo Scientific).

### Neutralization Assay

At 2 months from the final vaccination, mouse serum was collected for SARS-CoV-2 Neutralization Assay (Thermo Scientific). Briefly, the wells were washed 2 times with 1X Wash Buffer, then 100 µL of positive control or pre-diluted samples were added to the appropriate wells. 1X Assay Buffer was added for negative controls. The plate was covered and incubated for 30 minutes at room temperature (RT) with shaking. The wells were washed 3 additional times before 100 µL of 1X Biotin Conjugate solution was added into each well. The plate was covered and incubated for an additional 30 minutes at room temperature (RT) with shaking. After aspiration and washing, 1X Streptavidin-HRP Conjugate was added and incubated for 15 mins at RT, followed by 3 more times of washing. Then, 100 µL Substrate Solution was added to each well and incubated for 15 minutes at RT. Finally, 100 µL Stop Solution was added to each well. The solution in the wells changes 5 from blue to yellow. The absorbance collectively was read at 450 nm by a Varioskan Flash spectral scanning multimode reader (Thermo Scientific).

### Flow Cytometry

Murine subcutaneous tumors were processed into single-cell suspensions after passing through a 70-µm cell strainer (Thermo Fisher Scientific). At 4°C, 1-2 x 10⁶ cells were incubated with Block buffer (1 × PBS, 2% FBS, 0.5% BSA) for 15 minutes, prior to the addition of 50µl Master Mix surface marker staining (FITC anti-mouse CD3, PE/Cy7 anti-mouse CD4, BV-421 anti-mouse CD8a, 1:50, BioLegend) for 30 minutes. Cells were washed in filtered PBS and centrifuged at 2000 RPM before resuspension in 200 µl FACs buffer (1 × PBS, 2% FBS, 0.002% Sodium Azide). For fluorescence-activated cell sorting, a NovoCyte Quanteon Flow cytometer was used to analyze the samples.

### Statistical analysis

All results were plotted in Graphpad Prism 7 (GraphPad Software Inc). Statistical comparisons between groups were determined by an unpaired t-test using Prism 7. Mantel-Cox tests were used for the survival analysis. For all statistical tests, p < 0.05 was considered statistically significant.

All patents, patent applications, provisional applications, and publications referred to or cited herein are incorporated by reference in their entirety, including all figures and tables, to the extent they are not inconsistent with the explicit teachings of this specification.

Following are examples that illustrate procedures for practicing the invention. These examples should not be construed as limiting. All percentages are by weight and all solvent mixture proportions are by volume unless otherwise noted.

### EXAMPLE 1 - Selection and synthesis of phase-separating peptide coacervates for mRNA delivery.

Peptides containing unique spacer and sticker sequences form coacervates to encapsulate nucleic acids for drug delivery. Consequently, various peptide spacer (KssK and RssR) and sticker (FF, FW, WF, WW) amino acid sequences have been selected for chemical synthesis **(Figure 1A**). Under different conditions (PBS and DMEM), the "droplet-like" status formed specifically by the WWKssKWW motif at physiological temperature (37⁰C) was observed by light microscopy **(****Figure 1B**). The other similar amino acid sequences formed either aggregates or non-uniform droplets. The WWKssKWW droplets undergo dynamic fusion characteristics over a 75-seconds time-lapse, supporting its phase separation-like behavior at room temperature **(****Figure 1C**). WWKssKWW droplets could also encapsulate fluorescent nucleic acids by liquid-liquid phase separation **(****Figure 1D**). In addition, redox-based liquid chromatography performed on the WWKssKWW motif with ascending concentrations of GSH (1, 5, and 10mM) reveals the molecular separation of the coacervates under the liquid phase **(****Figures 1E** and **1F****).** Preparation of the phase-separating peptide mRNA formulation for the further *in vitro* and *in vivo* biological applications involves a fast and simple mixing procedure between the peptide coacervates and the mRNA at 37⁰C in DMEM, followed by a 15-minute incubation **(****Figure 1G****).**

### EXAMPLE 2 - In vitro delivery of luciferase mRNA using phase-separating peptides.

To validate the cellular delivery of luciferase mRNA by the phase-separating peptide (WWKssKWW motif), we performed a head-to-head comparison of the phase-separating peptide mRNA formulation with several commercially available mRNA transfection agents **(****Figure 3A****).** Initially, we performed *in vitro* transfection of mammalian HEK293 cells using various concentrations of WWKssKWW and analyzed the well-plate luminescence 24 hours after the transfection **(****Figure 3B****).** The empty vectors induced negligible luminescence. When the mRNA (1µg) was added, the phase-separating peptide mixtures could induce strong luminescence intensities which peaked at the peptide concentration of 0.9mg/ml **(****Figures 3C** and **8A****).** This peptide concentration was chosen as the optimal dose for further experiments. This data supported the cellular internalization and subsequent expression of luciferase mRNA. To examine the cytotoxicity of the phase-separating peptide mRNA formulations, we performed the cell viability assay with ascending doses of phase-separating peptide (0 to 1.2 mg/ml). All concentrations of WWKssKWW up to 0.9 mg/ml were well-tolerated and produced no significant difference in cell viability compared to healthy HEK293 cells **(****Figure 2D****).** When the concentration was further increased to 1.2 mg/ml, the cell viability dropped to approximately 70 percent. This might explain the reduced level of mRNA luminescence at higher concentrations of phase-separating peptide **(****Figures 3B** and **3C****).** For the *in vitro* transfection experiment, the optimal dose of the phase-separating peptide produced significantly stronger mRNA expression than the Lipofectamine^{™} MessengerMAX^{™}, InstantFECT cationic liposome, PEI, and protamine formulation **(****Figures 3E** and **3F****).** Naked mRNA alone also induced negligible luminescence **(****Figures 3F** and **8B****).** Consequently, the optimal phase-separating peptide (0.9 mg/ml) and mRNA (1µg) dose ratio were adopted for the next *in vivo* experiments **(****Figures 4A** and **4H****).** We have also investigated the *in vitro* transfection of DC2.4 cell lines. The phase-separating formulation yielded some mRNA expression from dendritic cells, unlike the Lipofectamine^{™} MessengerMAX^{™} formulation which produced no detectable luminescence **(****Figure 9****).**

### EXAMPLE 3 - In vivo delivery of luciferase mRNA using phase-separating peptides.

To investigate the *in vivo* expression of mRNAs delivered by the WWKssKWW peptide (10µg luciferase mRNA with 0.9 mg/mL peptide were injected into a mouse), we performed IVIS animal imaging 24 and 48 hours after the subcutaneous delivery or intramuscular delivery of various modified luciferase mRNA formulations **(****Figure 4A****).** The phase-separating peptide formulation allowed robust luciferase mRNA expression after subcutaneous and intramuscular injection **(****Figure 4B****).** At 24 hours, the phase-separating peptide mRNA subcutaneous formulation could induce significant levels of luminescence in mice compared to the PEI mRNA formulation, and vehicle controls, and comparable levels of luminescence with the Lipofectamine^{™} MessengerMAX^{™} mRNA formulation **(****Figures 4C** and **10****).** The InstantFECT mRNA formulation produced the highest luminescence at this time point. At 48 hours, the phase-separating peptide mRNA subcutaneous formulation produced comparable levels of luminescence in mice with the InstantFECT mRNA formulation, and significantly higher levels of luminescence than the Lipofectamine^{™} MessengerMAX^{™} mRNA formulation, PEI mRNA formulation, and vehicle controls **(****Figure 4D****).** Interestingly, the phase-separating peptide mRNA intramuscular formulation could generate greater luminescence in mice compared to the vehicle controls, PEI mRNA, and Lipofectamine^{™} MessengerMAX^{™} mRNA formulation at 24 hours **(****Figure 4E****).** The InstantFECT mRNA formulation produced comparable luminescence intensity with the phase-separating peptide mRNA formulation at this time point. At 48 hours, the luminescence levels between the phase-separating peptide mRNA, InstantFECT mRNA, and Lipofectamine^{™} MessengerMAX^{™} mRNA mice groups were comparable **(****Figure 4F****).** Regardless, the phase-separating peptide mRNA intramuscular formulation could generate greater luminescence in mice compared to the vehicle controls and PEI mRNA formulation. Altogether, these results indicate the sufficient *in vivo* expression of mRNA delivered by the WWKssKWW phase-separating peptide. Importantly, the mice did not exhibit any signs of weight loss, organ abnormalities, discomfort, or side effects after the phase-separating peptide injection **(****Figures 11****,** **12A,** and **12B****).** Unlike the other mRNA formulations, the subcutaneous and intramuscular injection of the PEI mRNA formulation led to severe discomfort and some mice died by the 24-hour time point. To further examine the potential *in vivo* toxicity of various mRNA formulations, we conducted a histological harvest of various mice organs at the 24-hour time point. The liver sections from InstantFECT mRNA and Lipofectamine^{™} MessengerMAX^{™} mRNA mice groups exhibited congestive hepatopathy, unlike the phase-separating peptide and healthy control animals **(****Figure 4G****).** Although most congestive hepatopathy cases due to lipid carriers are asymptomatic, some cases may cause abdominal discomfort⁴⁷⁻⁴⁹. No other histological organ differences were observed between the groups **(****Figure 4H****).** These findings support the safe utilization of phase-separating peptides to deliver mRNA vaccines.

### EXAMPLE 4 - Cellular immunogenicity of antigen mRNAs and TLR 7 peptide delivery by phase-separating peptides.

The antigen-specific cellular response towards biological vaccines could indicate the extent of immune protection. To study the immunogenicity of phase-separating peptide-delivered mRNA vaccines and phase-separating peptide-delivered adjuvants, we performed interferon-gamma ELISPOT on the isolated splenocytes of various subcutaneously immunized mice (phase-separating peptide OVA mRNA, Lipofectamine^{™} MessengerMAX^{™} OVA mRNA, phase-separating peptide OVA mRNA plus TLR7, phase-separating peptide TLR7, TLR7 alone, and vehicle controls **(****Figure 5A****).** Interestingly, the phase-separating peptide OVA mRNA could stimulate significantly greater OVA-specific cellular response than Lipofectamine^{™} MessengerMAX^{™} OVA mRNA and vehicle controls, whereas responses were comparable to those of the InstantFECT OVA mRNA **(****Figures 5B** and **5C****).** These findings support the enhanced cellular uptake of antigen mRNA vaccines delivered by the phase-separating peptide. Additionally, phase-separating peptides permitted TLR7 adjuvant delivery and the combined subcutaneous treatment with OVA mRNA could augment OVA-specific cellular responses in the immunized mice compared to the phase-separating peptide OVA mRNA alone **(****Figures 5D** and **5E****).** Phase-separating peptides also facilitated efficient intramuscular delivery of SARS-CoV-2 spike mRNA and produced comparable spike-specific cellular responses with the commercial Comirnaty mRNA vaccine **(****Figures 5F-5H**). The humoral response in the phase-separating peptide spike mRNA group at this dosage was adequate to induce anti-spike antibodies when compared with the vehicle control group, although levels were lower than those of the Comirnaty mRNA vaccine group **(****Figures 13A** and **13B****).** This result might implicate the differences in mRNA vaccine uptake mechanisms between the different mRNA carrier formulations. Overall, these results demonstrate potent antigen mRNA delivery by phase-separating peptides.

### EXAMPLE 5- Humoral immunogenicity of SARS-CoV-2 spike mRNA delivery by phase-separating peptides

The generation of humoral responses, via antigen-specific antibody production, could indicate immune protection against infectious disease, such as SARS-CoV-2. Previous data indicated that low dose spike mRNA (15 µg/mice) could elicit some antibody titers **(****Figures 13A** and **13B****).** Next, we sought to investigate whether greater immunogenicity could be established by enhancing the dosage of spike mRNA (25 µg/mice) delivered by phase-separating peptides via intramuscular injection at days 0 and 21 **(****Figure 6A****).** We performed serum ELISA at one and two months after immunization. To our surprise, the phase-separating peptide spike mRNA immunizations generated significantly high levels of spike specific IgG antibodies which are comparable to those of the InstantFECT spike mRNA group **(****Figures 6B** and **6C****).** Nonetheless, the Comirnaty COVID-19 mRNA vaccine produced the greatest number of antibodies **(****Figures 6B** and **6C****)** despite previously producing comparable levels of cellular immunity **(****Figures 5G** and **5H**). The heightened humoral response produced by Comirnaty could be due to the stable mRNA expression generated by the clinical preparation of spike mRNA and lipid nanoparticle formulations. Clinical development augments the stability of mRNA vaccines through the stringent monitoring of compound size, temperature, pH, aggregation, moiety modifications in the preparation stage¹⁹. Finally, the ratios of IgG1 and IgG2A amongst the antibodies generated by the different spike mRNA vaccines were comparable, which suggests equal levels of Th2-based and Th1-based immunity, respectively **(****Figure 6D****).** Neutralization antibodies against viral infections are a gold-standard marker of antiviral immune protection generated by vaccines. As expected, significant levels of neutralization antibodies against the WT, Delta, and Alpha SARS-CoV-2 were also generated by the phase-separating peptide spike mRNA immunizations which were comparable to the other treatment groups **(****Figures 6E** and **6G****).** Altogether, potent antigen-specific humoral immunity resulted from the intramuscular immunization of phase-separating peptide mRNA vaccines, which highlighted the promise of applying these vaccines in the context of infectious disease prevention.

### EXAMPLE 6 - Efficacy of phase-separating peptide OVA mRNA treatment against B16-OVA cancer.

The phase-separating peptide formulation can elicit stronger *in vitro* expression of luciferase mRNA than the commercially available mRNA transfection reagents. For *in vivo* applications, phase-separating peptide formulation can sufficiently deliver OVA mRNA, SARS-CoV-2 spike mRNA, and TLR7 adjuvants to induce cellular and humoral immune responses. As a cancer therapy, the phase-separating peptide OVA mRNA treatment effectively suppresses B16-OVA tumor growth and significantly improves the survival of tumor-bearing mice.

To test the therapeutic potential of phase-separating peptides for delivering mRNA vaccine payloads, we established a B16-OVA subcutaneous tumor model to compare the efficacy of various OVA mRNA formulations (phase-separating peptide OVA mRNA, InstantFECT mRNA, Lipofectamine^{™} MessengerMAX^{™} mRNA, Lipofectamine^{™} MessengerMAX^{™} control, InstantFECT control, and phase-separating peptide control) **(****Figure 7A****).** Outstandingly, the phase-separating peptide OVA mRNA group (average tumor size = 42.5 mm³) demonstrated significant tumor suppression compared to the Lipofectamine^{™} MessengerMAX^{™} mRNA (average tumor size = 292 mm³), Lipofectamine^{™} MessengerMAX^{™} control (average tumor size = 1256 mm³) and phase-separating peptide control (average tumor size = 1024 mm³) by the tumor endpoint **(****Figures 7B****,** **7H****,** and **7I****).** The tumor sizes and survival rates were comparable between the phase-separating peptide OVA mRNA group and the InstantFECT mRNA group **(****Figures 7I** and **7J****).** Subsequently, the phase-separating peptide OVA mRNA could significantly prolong the tumor-free mice survival rate (2/5 mice survived) compared to all vehicle controls (all mice died) by the day 40 humane euthanization endpoint **(****Figure 7J****).** Without being bound to any theory, tumor-infiltrating cytotoxic CD8+ T cells might exhibit potent tumor killing leading to the observed cancer eradication. Consequently, we conducted flow cytometry on the harvested tumor of various groups at the tumor endpoint. Significantly elevated levels of CD8+ T cells but not CD4+ T cells were detected in the phase-separating peptide OVA mRNA-treated tumors compared to the Lipofectamine^{™} MessengerMAX^{™} OVA mRNA-treated tumors and the vehicle control tumors **(****Figures 7K** and **7L****).** Altogether, these results demonstrate the robust efficacy of the phase-separating peptide OVA mRNA treatment and provide valuable insights regarding the establishment of peptide coacervates-based drug carriers in the field of cancer immunotherapy and mRNA drug delivery against various diseases.

It should be understood that the examples and embodiments described herein are for illustrative purposes only and that various modifications or changes in light thereof will be suggested to persons skilled in the art and are to be included within the spirit and purview of this application. In addition, any elements or limitations of any invention or embodiment thereof disclosed herein can be combined with any and/or all other elements or limitations (individually or in any combination) or any other invention or embodiment thereof disclosed herein, and all such combinations are contemplated with the scope of the invention without limitation thereto.

### EXEMPLARY EMBODIMENTS

Embodiment 1. A nucleic acid delivery system, comprising coacervate droplets formed by a phase-separating tripeptide dimer, wherein the phase-separating tripeptide dimer comprises a spacer having a disulfide-linked amino acid sequence KssK and two stickers having an amino acid sequence WW, wherein nucleic acids are encapsulated in the droplets, wherein the nucleic acids encode one or more vaccines or drugs.
Embodiment 2. The system of embodiment 1, wherein the phase-separating tripeptide dimer comprises the amino acid sequence WWKssKWW.
Embodiment 3. The system of embodiment 1 or 2, wherein the phase-separating tripeptide dimer comprises unnatural amino acids.
Embodiment 4. The system of any preceding embodiment, wherein the phase-separating tripeptide dimer comprises more than three amino acids in each monomer.
Embodiment 5. The system of any preceding embodiment, wherein the phase-separating tripeptide dimer utilizes other methods of glutathione-responsive release inside the cells other than the disulfide.
Embodiment 6. The system of any preceding embodiment, wherein a WWKssKWW coacervate in a buffer solution is in a droplet-like status at a temperature of about 37 °C.
Embodiment 7. The method of any preceding embodiment, wherein the nucleic acids are encapsulated in a WWKssKWW coacervate droplet.
Embodiment 8. A method for delivering a nucleic acid therapeutic inside a cell, comprising:
   (a) obtaining a phase-separating peptide;
   (b) generating a phase-separating peptide coacervate comprising the phase-separating peptide;
   (c) obtaining a nucleic acid sequence encoding a vaccine or drug;
   (d) mixing the nucleic acid with the phase-separating peptide coacervate to obtain a droplet that encapsulates the nucleic acid, and
   (e) administering a composition comprising the droplet to a subject whereby the nucleic acid is delivered inside a cell of the subject.
Embodiment 9. The method of embodiment 8, wherein the phase-separating peptide comprises the amino acid sequence WWKssKWW.
Embodiment 10. The method of embodiment 8 or 9, wherein the nucleic acid is an mRNA or DNA encoding a vaccine or drug, wherein the mRNA or DNA encoded vaccine or drug comprises one or more tumor antigens.
Embodiment 11. The method of any of embodiments 8-10, wherein the nucleic acid is an mRNA or DNA encoding a vaccine or drug, wherein the mRNA or DNA encoded vaccine or drug comprises one or more viral antigens.
Embodiment 12. The method of any of embodiments 8-11, wherein the mRNA or DNA encoded vaccine or drug comprises one or more SARS-CoV-2 spike antigens.
Embodiment 13. The method of any of embodiments 8-12, wherein the composition comprises an adjuvant.
Embodiment 14. The method of embodiment 13, wherein the adjuvant comprises TLR7.
Embodiment 15. The method of any of embodiments 8-14, wherein the composition comprises an excipient and/or carrier.
Embodiment 16. The method of any of embodiments 8-15, wherein the subject is a mammal, and optionally is a human.
Embodiment 17. The method of any of embodiments 8-16, wherein the mRNA or DNA encoded vaccine elicits a strong cellular interferon gamma response in the subject.
Embodiment 18. The method of any of embodiments 8-16, wherein the mRNA or DNA encoded vaccine elicits a strong humoral response in the subject.
Embodiment 19. The method of any of embodiments 8-18, wherein the mRNA or DNA encoded drug elicits a remission of the tumor.
Embodiment 20. The method of embodiment 19, wherein the mRNA or DNA encoded drug substantially promotes tumor free survival.

### REFERENCES

1. Li J, Zhang Y, Yang YG, Sun T. Advancing mRNA Therapeutics: The Role and Future of Nanoparticle Delivery Systems. Mol Pharm. 2024 Jul 2. doi: 10.1021/acs.molpharmaceut.4c00276. Epub ahead of print. PMID: 38953708.
2. Li B, Shao H, Gao L, Li H, Sheng H, Zhu L. Nano-drug co-delivery system of natural active ingredients and chemotherapy drugs for cancer treatment: a review. Drug Deliv. 2022 Dec;29(1):2130-2161. doi: 10.1080/10717544.2022.2094498. Erratum in: Drug Deliv. 2022 Dec;29(1):3051. doi: 10.1080/10717544.2022.2127213. PMID: 35815678; PMCID: PMC9275501.
3. Qamar Z, Qizilbash FF, Iqubal MK, Ali A, Narang JK, Ali J, Baboota S. Nano-Based Drug Delivery System: Recent Strategies for the Treatment of Ocular Disease and Future Perspective. Recent Pat Drug Deliv Formul. 2019;13(4):246-254. doi: 10.2174/1872211314666191224115211. PMID: 31884933; PMCID: PMC7499345.
4. Liang Y, Duan L, Lu J, Xia J. Engineering exosomes for targeted drug delivery. Theranostics. 2021 Jan 1;11(7):3183-3195. doi: 10.7150/thno.52570. PMID: 33537081; PMCID: PMC7847680.
5. Yu J, Kuwentrai C, Gong HR, Li R, Zhang BZ, Lin X, Wang X, Huang JD, Xu C. Intradermal delivery of mRNA using cryomicroneedles. Acta Biomater. 2022 Aug;148:133-141. doi: 10.1016/j.actbio.2022.06.015. Epub 2022 Jun 11. PMID: 35697200.
6. Lorentzen CL, Haanen JB, Met Ö, Svane IM. Clinical advances and ongoing trials on mRNA vaccines for cancer treatment. Lancet Oncol. 2022 Oct;23(10):e450-e458. doi: 10.1016/S1470-2045(22)00372-2. Erratum in: Lancet Oncol. 2022 Nov;23(11):e492. doi: 10.1016/S1470-2045(22)00608-8. PMID: 36174631; PMCID: PMC9512276.
7. Wang X, Yuen TT, Dou Y, Hu J, Li R, Zeng Z, Lin X, Gong H, Chan CH, Yoon C, Shuai H, Ho DT, Hung IF, Zhang BZ, Chu H, Huang JD. Vaccine-induced protection against SARS-CoV-2 requires IFN-γ-driven cellular immune response. Nat Commun. 2023 Jun 10;14(1):3440. doi: 10.1038/s41467-023-39096-y. PMID: 37301910; PMCID: PMC10257169.
8. Xu S, Yang K, Li R, Zhang L. mRNA Vaccine Era-Mechanisms, Drug Platform and Clinical Prospection. Int J Mol Sci. 2020 Sep 9;21(18):6582. doi: 10.3390/ijms21186582. PMID: 32916818; PMCID: PMC7554980.
9. Dong L, Deng X, Li Y, Zhu X, Shu M, Chen J, Luo H, An K, Cheng M, Zhang P, Tan W. Stimuli-Responsive mRNA Vaccines to Induce Robust CD8+ T Cell Response via ROS-Mediated Innate Immunity Boosting. J Am Chem Soc. 2024 Jul 2. doi: 10.1021/jacs.4c04331. Epub ahead of print. PMID: 38955767.
10. Kon E, Elia U, Peer D. Principles for designing an optimal mRNA lipid nanoparticle vaccine. Curr Opin Biotechnol. 2022 Feb;73:329-336. doi: 10.1016/j.copbio.2021.09.016. Epub 2021 Oct 26. PMID: 34715546; PMCID: PMC8547895.
11. Arevalo CP, Bolton MJ, Le Sage V, Ye N, Furey C, Muramatsu H, Alameh MG, Pardi N, Drapeau EM, Parkhouse K, Garretson T, Morris JS, Moncla LH, Tam YK, Fan SHY, Lakdawala SS, Weissman D, Hensley SE. A multivalent nucleoside-modified mRNA vaccine against all known influenza virus subtypes. Science. 2022 Nov 25;378(6622):899-904. doi: 10.1126/science.abm0271. Epub 2022 Nov 24. PMID: 36423275; PMCID: PMC10790309.
12. Crommelin DJA, Anchordoquy TJ, Volkin DB, Jiskoot W, Mastrobattista E. Addressing the Cold Reality of mRNA Vaccine Stability. J Pharm Sci. 2021 Mar;110(3):997-1001. doi: 10.1016/j.xphs.2020.12.006. Epub 2020 Dec 13. PMID: 33321139; PMCID: PMC7834447.
13. Bird L. mRNA vaccine for treating pancreatic cancer. Nat Rev Immunol. 2023 Jul;23(7):413. doi: 10.1038/s41577-023-00899-1. PMID: 37268820.
14. Tan T, Deng ST, Wu BH, Yang Q, Wu MW, Wu H, Cao CH, Xu C. mRNA Vaccine - A New Cancer Treatment Strategy. Curr Cancer Drug Targets. 2023;23(9):669-681. doi: 10.2174/1568009623666230222124424. PMID: 36809966.
15. Vishweshwaraiah YL, Dokholyan NV. mRNA vaccines for cancer immunotherapy. Front Immunol. 2022 Dec 14;13:1029069. doi: 10.3389/fimmu.2022.1029069. PMID: 36591226; PMCID: PMC9794995.
16. Ghasemiyeh P, Mohammadi-Samani S. Solid lipid nanoparticles and nanostructured lipid carriers as novel drug delivery systems: applications, advantages and disadvantages. Res Pharm Sci. 2018 Aug;13(4):288-303. doi: 10.4103/1735-5362.235156. PMID: 30065762; PMCID: PMC6040163.
17. Jin Y, Hou C, Li Y, Zheng K, Wang C. mRNA Vaccine: How to Meet the Challenge of SARS-CoV-2. Front Immunol. 2022 Jan 21;12:821538. doi: 10.3389/fimmu.2021.821538. PMID: 35126377; PMCID: PMC8813741.
18. Zhang A, Ji Q, Sheng X, Wu H. mRNA vaccine in gastrointestinal tumors: Immunomodulatory effects and immunotherapy. Biomed Pharmacother. 2023 Oct;166:115361. doi: 10.1016/j.biopha.2023.115361. Epub 2023 Sep 4. PMID: 37660645.
19. Yasmin F, Najeeb H, Naeem U, Moeed A, Atif AR, Asghar MS, Nimri N, Saleem M, Bandyopadhyay D, Krittanawong C, Fadelallah Eljack MM, Tahir MJ, Waqar F. Adverse events following COVID-19 mRNA vaccines: A systematic review of cardiovascular complication, thrombosis, and thrombocytopenia. Immun Inflamm Dis. 2023 Mar;11(3):e807. doi: 10.1002/iid3.807. PMID: 36988252; PMCID: PMC10022421.
20. Das, S., Vera, M., Gandin, V. et al. Intracellular mRNA transport and localized translation. Nat Rev Mol Cell Biol 22, 483-504 (2021). See worldwide website: doi.org/10.1038/s41580-021-00356-8
21. Wu B, Eliscovich C, Yoon YJ, Singer RH. Translation dynamics of single mRNAs in live cells and neurons. Science. 2016 Jun 17;352(6292):1430-5. doi: 10.1126/science.aaf1084. Epub 2016 May 5. PMID: 27313041; PMCID: PMC4939616.
22. Pardi, N., Hogan, M., Porter, F. et al. mRNA vaccines - a new era in vaccinology. Nat Rev Drug Discov 17, 261-279 (2018). See worldwide website: doi.org/10.1038/nrd.2017.243
23. Rishi R. Goel et al. mRNA vaccines induce durable immune memory to SARS-CoV-2 and variants of concern.Science374,abm0829(2021).DOI:10.1 126/science.abm0829
24. Verbeke R, Lentacker I, De Smedt SC, Dewitte H. The dawn of mRNA vaccines: The COVID-19 case. J Control Release. 2021 May 10;333:511-520. doi: 10.1016/j.jconrel.2021.03.043. Epub 2021 Mar 30. PMID: 33798667; PMCID: PMC8008785.
25. Biggs AT, Littlejohn LF. Describing mRNA Vaccine Technology for a Military Audience. Mil Med. 2023 Mar 20;188(3-4):547-554. doi: 10.1093/milmed/usac129. PMID: 35584186.
26. Biggs AT, Littlejohn LF. Describing mRNA Vaccine Technology for a Military Audience. Mil Med. 2023 Mar 20;188(3-4):547-554. doi: 10.1093/milmed/usac129. PMID: 35584186.
27. Cerrato CP, Leppert A, Sun Y, Lane DP, Arsenian-Henriksson M, Miserez A, Landreh M. Monitoring Disassembly and Cargo Release of Phase-Separated Peptide Coacervates with Native Mass Spectrometry. Anal Chem. 2023 Jul 25;95(29):10869-10872. doi: 10.1021/acs.analchem.3c02384. Epub 2023 Jul 13. PMID: 37439740; PMCID: PMC10372869.
28. Sun, Y., Lau, S.Y., Lim, Z.W. et al. Phase-separating peptides for direct cytosolic delivery and redox-activated release of macromolecular therapeutics. Nat. Chem. 14, 274-283 (2022). See worldwide website: doi.org/10.1038/s41557-021-00854-4
29. Anne Ramat, Martine Simonelig, Activating translation with phase separation.Science377,712-713(2022).DOI:10.1126/science.add6323
30. Bao Y, Chen H, Xu Z, Gao J, Jiang L, Xia J. Photo-Responsive Phase-Separating Fluorescent Molecules for Intracellular Protein Delivery. Angew Chem Int Ed Engl. 2023 Oct 16;62(42):e202307045. doi: 10.1002/anie.202307045. Epub 2023 Sep 12. PMID: 37648812.
31. Ladbury JE, Lin CC, Suen KM. Phase separation enhances probability of receptor signalling and drug targeting. Trends Biochem Sci. 2023 May;48(5):428-436. doi: 10.1016/j.tibs.2023.01.005. Epub 2023 Feb 7. PMID: 36759237.
32. Zhang B, Liu B, Wu Z, Oyama K, Ikari M, Yagi H, Tochio N, Kigawa T, Mikawa T, Miyake T. A Hybrid Nanotube Stamp System in Intracellular Protein Delivery for Cancer Treatment and NMR Analytical Techniques. Anal Chem. 2024 May 28;96(21):8349-8355. doi: 10.1021/acs.analchem.3c05331. Epub 2024 May 14. PMID: 38745349.
33. Gabryelczyk B, Alag R, Philips M, Low K, Venkatraman A, Kannaian B, Shi X, Linder M, Pervushin K, Miserez A. In vivo liquid-liquid phase separation protects amyloidogenic and aggregation-prone peptides during overexpression in Escherichia coli. Protein Sci. 2022 May;31(5):e4292. doi: 10.1002/pro.4292. PMID: 35481658; PMCID: PMC8994509.
34. Hyman AA, Weber CA, Jülicher F. Liquid-liquid phase separation in biology. Annu Rev Cell Dev Biol. 2014;30:39-58. doi: 10.1146/annurev-cellbio-100913-013325. PMID: 25288112.
35. Mittag T, Pappu RV. A conceptual framework for understanding phase separation and addressing open questions and challenges. Mol Cell. 2022 Jun 16;82(12):2201-2214. doi: 10.1016/j.molcel.2022.05.018. Epub 2022 Jun 7. PMID: 35675815; PMCID: PMC9233049.
36. Green CM, Sementa D, Mathur D, Melinger JS, Deshpande P, Elbaum-Garfinkle S, Medintz IL, Ulijn RV, Diaz SA. Sequestration within peptide coacervates improves the fluorescence intensity, kinetics, and limits of detection of dye-based DNA biosensors. Commun Chem. 2024 Feb 29;7(1):49. doi: 10.1038/s42004-024-01124-3. PMID: 38424154; PMCID: PMC10904739.
37. Shin Y. Rich Phase Separation Behavior of Biomolecules. Mol Cells. 2022 Jan 31;45(1):6-15. doi: 10.14348/molcells.2021.0204. PMID: 34966005; PMCID: PMC8819493.
38. Wen Y, Ma J. Phase separation drives the formation of biomolecular condensates in the immune system. Front Immunol. 2022 Nov 10;13:986589. doi: 10.3389/fimmu.2022.986589. PMID: 36439121; PMCID: PMC9685520.
39. Luo Y, Xiang S, Feng J. Protein Phase Separation: New Insights into Carcinogenesis. Cancers (Basel). 2022 Dec 2;14(23):5971. doi: 10.3390/cancers14235971. PMID: 36497453; PMCID: PMC9740862.
40. Luo Y, Xiang S, Feng J. Protein Phase Separation: New Insights into Carcinogenesis. Cancers (Basel). 2022 Dec 2;14(23):5971. doi: 10.3390/cancers14235971. PMID: 36497453; PMCID: PMC9740862.
41. Chen J, Ye Z, Huang C, Qiu M, Song D, Li Y, Xu Q. Lipid nanoparticle-mediated lymph node-targeting delivery of mRNA cancer vaccine elicits robust CD8+ T cell response. Proc Natl Acad Sci U S A. 2022 Aug 23;119(34):e2207841119. doi: 10.1073/pnas.2207841119. Epub 2022 Aug 15. PMID: 35969778; PMCID: PMC9407666.
42. Kowalski PS, Rudra A, Miao L, Anderson DG. Delivering the Messenger: Advances in Technologies for Therapeutic mRNA Delivery. Mol Ther. 2019 Apr 10;27(4):710-728. doi: 10.1016/j.ymthe.2019.02.012. Epub 2019 Feb 19. PMID: 30846391; PMCID: PMC6453548.
43. Qiu M, Li Y, Bloomer H, Xu Q. Developing Biodegradable Lipid Nanoparticles for Intracellular mRNA Delivery and Genome Editing. Acc Chem Res. 2021 Nov 2;54(21):4001-4011. doi: 10.1021/acs.accounts.1c00500. Epub 2021 Oct 20. PMID: 34668716.
44. Yang S, Yu H, Xu X, Yang T, Wei Y, Zan R, Zhang X, Ma Q, Shum HC, Song Y. AIEgen-Conjugated Phase-Separating Peptides Illuminate Intracellular RNA through Coacervation-Induced Emission. ACS Nano. 2023 May 9;17(9):8195-8203. doi: 10.1021/acsnano.2c12072. Epub 2023 Apr 24. PMID: 37093110.
45. Ries RJ, Zaccara S, Klein P, Olarerin-George A, Namkoong S, Pickering BF, Patil DP, Kwak H, Lee JH, Jaffrey SR. m6A enhances the phase separation potential of mRNA. Nature. 2019 Jul;571(7765):424-428. doi: 10.1038/s41586-019-1374-1. Epub 2019 Jul 10. PMID: 31292544; PMCID: PMC6662915.
46. Sun Y, Xu X, Chen L, Chew WL, Ping Y, Miserez A. Redox-Responsive Phase-Separating Peptide as a Universal Delivery Vehicle for CRISPR/Cas9 Genome Editing Machinery. ACS Nano. 2023 Sep 12;17(17):16597-16606. doi: 10.1021/acsnano.3c02669. Epub 2023 Aug 16. PMID: 37584415.
47. Mendonça MCP, Radaic A, Garcia-Fossa F, da Cruz-Höfling MA, Vinolo MAR, de Jesus MB. The in vivo toxicological profile of cationic solid lipid nanoparticles. Drug Deliv Transl Res. 2020 Feb;10(1):34-42. doi: 10.1007/s13346-019-00657-8. PMID: 31240624.
48. Husain H, Latief U, Ahmad R. Pomegranate action in curbing the incidence of liver injury triggered by Diethylnitrosamine by declining oxidative stress via Nrf2 and NFκB regulation. Sci Rep. 2018 Jun 5;8(1):8606. doi: 10.1038/s41598-018-26611-1. PMID: 29872102; PMCID: PMC5988808.
49. Yoon, H., Son, S., Lee, S. et al. Glycol chitosan nanoparticles as specialized cancer therapeutic vehicles: Sequential delivery of doxorubicin and Bcl-2 siRNA. Sci Rep 4, 6878 (2014). See worldwide website: doi.org/10.1038/srep06878
50. WO2021213945A1 - Coronavirus vaccine - Google Patents

## Claims

1. A nucleic acid delivery system, comprising coacervate droplets formed by a phase-separating tripeptide dimer, wherein the phase-separating tripeptide dimer comprises a spacer having a disulfide-linked amino acid sequence KssK and two stickers having an amino acid sequence WW, wherein nucleic acids are encapsulated in the droplets, wherein the nucleic acids encode one or more vaccines or drugs.

2. The system of claim 1, wherein the phase-separating tripeptide dimer comprises the amino acid sequence WWKssKWW,unnatural amino acids, and/ormore than three amino acids in each monomer.

3. The system of claim 1, wherein the phase-separating tripeptide dimer utilizes other methods of glutathione-responsive release inside the cells other than the disulfide.

4. The system of claim 1, wherein a WWKssKWW coacervate in a buffer solution is in a droplet-like status at a temperature of about 37 °C.

5. The system of claim 2, wherein the nucleic acids are encapsulated in a WWKssKWW coacervate droplet.

6. A method for delivering a nucleic acid therapeutic inside a cell, comprising:
(a) obtaining a phase-separating peptide;
(b) generating a phase-separating peptide coacervate comprising the phase-separating peptide;
(c) obtaining a nucleic acid sequence encoding a vaccine or drug;
(d) mixing the nucleic acid with the phase-separating peptide coacervate to obtain a droplet that encapsulates the nucleic acid, and
(e) administering a composition comprising the droplet to a subject whereby the nucleic acid is delivered inside a cell of the subject.

7. The method of claim 6, wherein the phase-separating peptide comprises an amino acid sequence WWKssKWW.

8. The method of claim 6, wherein the nucleic acid is an mRNA or DNA encoding a vaccine or drug, wherein the mRNA or DNA encoded vaccine or drug comprises one or more tumor antigens.

9. The method of claim 6, wherein the nucleic acid is an mRNA or DNA encoding a vaccine or drug, wherein the RNA or DNA encoded vaccine or drug comprises one or more viral antigens, optionally wherein the mRNA or DNA encoded vaccine or drug comprises one or more SARS-CoV-2 spike antigens.

10. The method of claim 6, wherein the composition comprises an excipient and/or carrier and/or an adjuvant, optionally wherein the adjuvant comprises TLR7.

11. The method of claim 6, wherein the subject is a mammal, and optionally is a human.

12. The method of claim 9, wherein the mRNA or DNA encoded vaccine elicits a strong cellular interferon gamma response in the subject.

13. The method of claim 8, wherein the mRNA or DNA encoded vaccine elicits a strong humoral response in the subject.

14. The method of claim 9, wherein the mRNA or DNA encoded drug elicits a remission of the tumor.

15. The method of claim 14, wherein the mRNA or DNA encoded drug substantially promotes tumor free survival.
